# EUROPÄISCHE PATENTSCHRIFT

(11) **EP 2 935 621 B1**
(45) Veröffentlichungstag und Bekanntmachung des Hinweises auf die Patenterteilung: **10.11.2021**
(21) Anmeldenummer: 13818717.4
(22) Anmeldetag: 17.12.2013
(51) Int. Cl.: C12Q 1/68

(54) **VERFAHREN ZUR BESTIUMMUNG DES DNA-METHYLIERUNGSGRADS**
METHOD FOR DETERMINING THE DEGREE OF DNA METHYLATION
PROCÉDÉ DE DÉTERMINATION DU DEGRÉ D'ALLIAGE ADN-MÉTHYLE

(30) Priorität: 21.12.2012 EP 12199152
(43) Veröffentlichungstag der Anmeldung: 28.10.2015
(60) Teilanmeldung: 21200307.3
(73) Patentinhaber: Santourlidis, Simeon, 40670 Meerbusch (DE)
(72) Erfinder: SANTOURLIDIS, Simeon, 40670 Meerbusch (DE); ARAUZO BRAVO, Marcos, 20009 San Sebastian-Donostia (ES); GHANJATI, Foued, 40591 Düsseldorf (DE); BEERMANN, Agnes, 46562 Voerde (DE); WERNET, Peter, 40627 Düsseldorf (DE); ACKERMANN, Rolf, 40589 Düsseldorf (DE)
(74) Vertreter: Cohausz & Florack
(86) Internationale Anmeldenummer: PCT/EP2013/076992
(87) Internationale Veröffentlichungsnummer: WO 2014/095933

(56) Entgegenhaltungen:
- WO-A2-2010/084154
- US-A1- 2008 260 743
- US-A1- 2011 269 132
- CHRISTINA DAHL ET AL: "DNA methylation analysis techniques", BIOGERONTOLOGY, KLUWER ACADEMIC PUBLISHERS, DO, Bd. 4, Nr. 4, 1. August 2003 (2003-08-01), Seiten 233-250, XP019230475, ISSN: 1573-6768, DOI: 10.1023/A:1025103319328
- RAJU KANDIMALLA ET AL: "Genome-wide Analysis of CpG Island Methylation in Bladder Cancer Identifiedandas pTa-Specific Prognostic Markers", EUROPEAN UROLOGY, ELSEVIER BV, NL, Bd. 61, Nr. 6, 9. Januar 2012 (2012-01-09) , Seiten 1245-1256, XP028419471, ISSN: 0302-2838, DOI: 10.1016/J.EURURO.2012.01.011 [gefunden am 2012-01-10]
- S. T. BORNO ET AL: "Genome-wide DNA Methylation Events in TMPRSS2-ERG Fusion-Negative Prostate Cancers Implicate an EZH2-Dependent Mechanism with miR-26a Hypermethylation", CANCER DISCOVERY, Bd. 2, Nr. 11, 28. August 2012 (2012-08-28), Seiten 1024-1035, XP055106290, ISSN: 2159-8274, DOI: 10.1158/2159-8290.CD-12-0041
- TRINH BINH N ET AL: "DNA methylation analysis by MethyLight technology", METHODS : A COMPANION TO METHODS IN ENZYMOLOGY, ACADEMIC PRESS INC., NEW YORK, NY, US, Bd. 25, Nr. 4, 1. Dezember 2001 (2001-12-01), Seiten 456-462, XP002318911, ISSN: 1046-2023, DOI: 10.1006/METH.2001.1268
- None

## Beschreibung

Die Erfindung betrifft ein Verfahren zur Bestimmung des normierten DNA-Methylierungsgrads eines DNA-Abschnitts.

Das Genom eukaryotischer Zellen besitzt ein DNA-Methylierungsmuster, welches wesentlich für die Kontrolle der Genexpression ist. Die häufigste Methylierung ist die Methylierung von Cytosin zum 5-Methyl-Cytosin.

Grundsätzlich deutet eine geringe Methylierung daraufhin, dass die entsprechende genomische Region aktiv transkribiert wird, wohingegen methylierte Abschnitte zumeist inaktiv sind. Es ist allgemein anerkannt, dass die Analyse epigenetischer Veränderungen ein diagnostisches und prognostisches Potential bei einer Vielzahl von Erkrankungen, insbesondere von Tumorerkrankungen, bietet.

Die WO 2008/149237 A2 beschreibt ein Verfahren zu Methylierungsanalyse und seine Anwendung insbesondere hinsichtlich des Septin 9-Gens. Zur Quantifizierung wurden genomische DNAs als Standards eingesetzt.

Die WO 2005/075671 A1 betrifft Verfahren zur Kalibrierung und Kontrolle von Methylierungsanalysen. Hierbei wird zur Kalibrierung des Assays komplett methylierte als auch nicht methylierte DNA eingesetzt; dabei wird die vollständig methylierte DNA beispielsweise durch Behandlung mit SssI-Methylase und die nicht methylierte DNA durch WGA (whole genome amplification) hergestellt. Dieses Kalibrationsverfahren ist weit verbreitet, entsprechende nicht-methylierte und vollständig methylierte DNA ist kommerziell erhältlich und wird häufig als Standard verwendet. Es handelt sich hierbei um positive und negative Referenzproben, auf die eine ggf. intermediäre DNA-Methylierung der Messproben bezogen werden kann, um diese relativ quantifizieren zu können.

Die WO 2010/084154 A2 beschreibt ein Verfahren zur Normierung des Methylierungsgrads von Transposons und deren Fragmenten im Genom. Dieses Verfahren ermöglicht wegen der Transposons als Ziel und deren einheitlichen Methylierungsstatus eine Angabe zum Methylierungsgrad, die repräsentativ für das gesamte Genom ist. Diese Angabe ist vorteilhaft, wenn eine Analyse der Änderung der DNA-Methylierung in einer Richtung über das gesamte Genom im Vordergrund steht. Die Methylierung ist entweder vermindert oder unverändert. Es wird immer dieselbe Transposon-Sequenz abgegriffen. Letztlich kann nur eine Aussage darüber getroffen werden, dass beispielsweise im Fall der Line 1-Promotorsequenz diese vermindert oder unverändert methyliert ist. Die verminderte Methylierung kann bereits in frühen Phasen der Tumorentstehung auftreten, so dass sich mit dem bekannten Verfahren ein Tumor bereits in einem frühen Stadium nachweisen läßt. Hypermethylierte Line 1-Sequenzen kommen in einem Tumor nicht vor.

CANCER DISCOVERY, 2012; 2:1024-1035 beschreibt ein Hochdurchsatz-Sequenzierverfahren zur Untersuchung des genom-weiten Methylierungspatterns in einer Kohorte von 51 Proben von Prostata-Tumor und 53 Proben von benigner Prostata-Hyperplasie offenbart wird, wobei Immunopräzipitate methylierter DNA sequenziert wurden. In Vergleichsuntersuchungen wurden eine große Zahl Krebsassoziierter epigenetischer Änderungen identifiziert. Es wird beschrieben, dass differenzielle Methylierung alternativ zur Prognose der Tumorentwicklung herangezogen werden kann. Die US 2010/ 0029009A1 beschreibt ein Verfahren zur Bestimmung des normierten DNA-Methylierungsgrads spezifischer genomischer Strukturen in einer aus peripheren Lymphozyten isolierten DNA. Die US 2008/0260743 A1 beschreibt eine Nukleinsäuremolekül mit einer Nukleotidsequenz, die ein bifunktionales Polypeptid kodiert, das eine DNA-Bindungsdomain für ein Protein, das zu Familie der Methyl-CpG bindenden Proteine /MBDs) gehört, und einen Fc-Teil eines Antikörpers enthält. EUROPEAN UROLOGY 61 (2012) 1245-1256 befasst sich mit Folgen epigenetischer Modifikationen der DNA und deren Beitrag zum Harnblasenkarzinom.

Aufgabe der Erfindung ist es, ein diagnostisches Verfahren bereitzustellen, das unter Berücksichtigung der differentiellen Methylierung von Gen-Abschnitten eine Aussage über das Stadium einer Tumorerkrankung ermöglicht.

Gelöst wird die Aufgabe durch ein Verfahren zur Bestimmung des relativen DNA-Methylierungsgrads, umfassend die Schritte
(i) Bestimmung des normierten Methylierungsgrads eines oder mehrerer DNA-Abschnitts/e, ausgenommen repetitive Abschnitte, in einer Probe genomischer DNA, umfassend die Schritte
   a) Quantitative Bestimmung der Gegenwart des DNA-Abschnitts in der Probe;
   b) Quantitative Bestimmung des Vorliegens von zumindest einem differentiell methylierten C eines CpG Dinukleotids innerhalb des DNA-Abschnitts und
   c) Bestimmung des normierten DNA-Methylierungsgrads des DNA-Abschnitts über die in den Schritten a) und b) bestimmten Werte für eine erste genomische DNA und eine zweite genomische DNA, wobei der DNA-Abschnitt ausgewählt ist aus einem oder mehreren der folgenden Gene:
      FAM50B-Ups, PITPNM1-Ups, LOC100419707-Cor, NTAN1-Cor, RPL36AP39-Ups, MIR324-Ups, HMGN2P28-Ups, MIR5696-Cor, HMGN2P28-Cor, RPS27P20-Cor, MPP7-Ups, MYEF2-Ups, TMEM254-Ups, VWC2L-Ups, CFHR5-Cor, ZSCAN1-Cor, RPL3P10-Ups, MYEF2-Cor, TMSB4XP2-Cor, SCN8A-Ups, PPDPF-Cor, TRNAQ15-Cor, TCTN2-Cor, RAD21L1-Cor, JOSD2-Ups, ZNF586-Ups, C10orf47-Cor, KIR2DP1-Cor, MAGEB1-Ups, IFNA17-Ups, TRIM62-Ups, MAGEB1-Cor, CTSW-Ups, LOC100507165-Cor, MIR4692-Ups, PLIN2-Cor, RPL7AP64-Cor, HIGD1AP12-Cor, MST1-Cor, ITGAD-Ups, DAK-Cor, FAM66E-Cor, HMGB3-Ups, FAM90A24P-Cor, LOC283335-Cor, MIR338-Cor, ADORA2BP1-Cor, CALD1-Ups, POU5F1P6-Ups, ARHGEF1-Cor, CNFN-Ups, MAST1-Ups, SCDP1-Ups, OR6C2-Ups, HOXB-AS1-Cor, PPIGP1-Ups, RMI2-Cor, TIGD1-Cor,
      BRE-AS1-Cor, MIRLET7F2-Cor,
      RNASEH2CP1-Cor, FAM90A24P-Ups, NAV2-AS5-Ups, SEPT3-Cor, RPS15AP36-Cor, KLRC1-Ups, KRT23-Ups,
      RPL13P7-Ups, LOC100128048-Cor, OR4A42P-Cor, LOC100996515-Ups, ARHGEF39-Cor, CYP3A4-Ups, MTND5P20-Cor, TSGA10IP-Ups, RBBP9-Cor, RPL39P25-Cor, NTMT1-Cor, LOC101060199-Cor, ZNF491-Cor, CIT-Cor, TPTE2P3-Ups, LOC338817-Cor, AK3P3-Ups, SEPT3-Cor, LOC126860-Cor,
(ii) Bestimmung des relativen DNA-Methylierungsgrads über das Verhältnis der für die erste genomische DNA und die zweite genomische DNA bestimmten normierten Methylierungsgrade,
   zur Diagnose einer Erkrankung, die mit einer veränderten DNA-Methylierung in Verbindung gebracht wird, wobei der relative Methylierungsgrad eine Hypermethylierung bedeutet und wobei die erste genomische DNA eine Referenzprobe ist und die zweite genomische DNA aus einer zu untersuchenden Probe stammt und die Erkrankung ein Prostata-Tumor ist.

Gegenstand der Erfindung ist auch ein Verfahren zur Bestimmung des relativen DNA-Methylierungsgrads, umfassend die Schritte
(i) Bestimmung des normierten Methylierungsgrads eines oder mehrerer DNA-Abschnitts/e, ausgenommen repetitive Abschnitte, in einer Probe genomischer DNA, umfassend die Schritte:
   a) Quantitative Bestimmung der Gegenwart des DNA-Abschnitts in der Probe;
   b) Quantitative Bestimmung des Vorliegens von zumindest einem differentiell methylierten C eines CpG Dinukleotids innerhalb des DNA-Abschnitts und
   c) Bestimmung des normierten DNA-Methylierungsgrads des DNA-Abschnitts über die in den Schritten a) und b) bestimmten Werte für eine erste genomische DNA und eine zweite genomische DNA, wobei der DNA-Abschnitt ausgewählt ist aus einem oder mehreren der folgenden Gene:
      WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, LOC401317-Cor, CRNKL1-Ups, MCCD1P2-Ups, DSG1-Cor, SLC22A24-Ups, ZNF26-Cor, NXPH4-Cor, MEN1-Ups, KDM3A-Cor, ARHGAP35-Cor, TRNAA2-Ups, EIF1AD-Ups, ACTN3-Cor, SPTY2D1-Cor, ASCL2-Ups, ZNF132-Cor, HMOX1-Cor, PPP1R2-Ups, WBSCR17-Cor, ARSH-Cor, ALDH7A1P3-Cor, NAGLU-Ups, ACRBP-Cor, ZNF304-Ups, MIR1972-2-Ups, TMEM33-Ups, LOC100419839-Ups, C1R-Cor, SNORD88B-Cor, NDUFB7-Cor, NTMT1-Ups, BRD4-Cor, LOC100131510-Cor, TIMM8BP2-Ups, KCNK1-Cor, RIMKLA-Cor, RPL12P42-Ups, PCGF6-Cor, LOC100419895-Cor, IZUMO2-Ups, PCBP3-Ups, ASS1P13-Cor, LOC100419848-Ups, LZTS1-AS1-Cor, LOC145757-Cor, PPFIA3-Cor, TRNAV20-Ups, LARP1P1-Cor, ZNF187-Ups, MIR4518-Cor, LOC100130394-Ups, ACPT-Cor, RPL23AP18-Cor, ODF2L-Cor, C16orf46-Ups, LOC100422241-Ups,
      ZHX3-Ups, RPL23AP32-Ups, ANKRD12-Cor, CD27-AS1-Cor, TPI1-Ups, KCNG4-Ups, GUSBP5-Ups, LOC147670-Cor, HYDIN-Cor, PKD2L1-Ups, GRIFIN-Ups, MIR548AK-Cor, YBX2-Cor, HNRNPA3P4-Ups, ATG2A-Cor, CDC42EP5-Ups, HBG2-Ups, DDB1-Cor, FAM90A11P-Cor, RPL9P17-Ups, TBX5-AS1-Ups, ZNF415-Cor, AURKC-Ups, EPHA10-Cor, KBTBD11-Ups, LOC100996788-Ups, ORAI3-Ups, LOC100507530-Ups, VSTM2L-Ups, ACTR5-Cor, LOC284080-Cor, LOC100996366-Cor, MIR526A2-Cor, MIR516B2-Cor, LOC100130632-Cor, CIB3-Cor, CHST9-Cor, ZNF788-Ups, ANKRD16-Cor, EIF4E3-Cor, CTXN2-Cor, LOC100631378-Cor, RPS2P48-Cor, FAM87B-Ups, LOC644881-Cor, TRNAS7-Ups, LSMD1-Ups,
      LOC390203-Ups, ARPC4-Cor, TAF6L-Ups, PROCR-Ups, RNU5B-4P-Cor, MIR548A2-Ups,-ASCL2-Cor, FOXRED1-Cor, MAPK8IP3-Cor, PIM1-Cor, HMGN2P2-Ups, MIR3972-Ups, LHFPL3-AS1-Ups, ZNF528-Ups, RPS15AP32-Ups, MIR516B2-Ups, FUT5-Cor, GPX1-Cor, C7orf65-Cor, RNY5P6-Ups, SYDE1-Cor, LOC389120-Ups, CDK2AP1-Cor, MIR518B-Cor, LOC100506753-Cor, REP15-Ups, EIF4G3-Ups, HIATL2-Ups, ZNF534-Cor, LOC115110-Cor, ATXN7L1-Ups, LRRC3C-Ups, VTI1BP3-Cor, LOC100507206-Ups, PUS1-Cor, SRP14P1-Ups, ODC1-Ups, RPL30P4-Ups, ZNF229-Cor, SIGLEC7-Ups, LOC654780-Cor, TPBGL-Cor,
      EHD1-Ups, TCEB3-Cor, KCNG4-Cor, SNORD5-Cor, CTXN2-Cor, PITPNM1-Ups, LDHB-Cor, LY6G5C-Ups, ID3-Cor, PELI3-Cor, MIR526A2-Cor, RPS29P24-Ups, IMPDH1P5-Ups, VWC2L-Cor, FAM71E2-Ups, RPL21P4-Ups, KCNQ4-Ups, ZNF512-Cor, ANKRD16-Cor, DHX8-Cor, STARD3-Cor, ZSCAN23-Ups, LOC100130632-Cor, KLF2-Cor, VSTM5-Ups, LOC151174-Cor, PGBD1-Ups, APPL1-Ups, COX6A1P2-Ups, ZNF775-Ups, SLC16A6-Cor, RPL9P29-Cor, IFT74-Cor, LOC100420668-Cor, IGSF21-Ups, PCBP3-Cor,
(ii) Bestimmung des relativen DNA-Methylierungsgrads über das Verhältnis der für die erste und die zweite DNA bestimmten Methylierungsgrade, zur Diagnose einer Erkrankung, die mit einer veränderten DNA-Methylierung in Verbindung gebracht wird, wobei der relative Methylierungsgrad eine Hypomethylierung bedeutet und wobei die erste genomische DNA eine Referenzprobe ist und die zweite genomische DNA aus einer zu untersuchenden Probe stammt und die Erkrankung ein Prostata-Tumor ist.

Dabei können Schritt a) und b) in beliebiger Reihenfolge oder auch gleichzeitig erfolgen.

Überraschend wurden Gen-Abschnitte aufgefunden, deren differentielle Methylierung einen Hinweis auf eine Tumorerkrankung und deren Status anzeigt. Insbesondere wurde erfindungsgemäß festgestellt, dass die Zusammenschau der differentiellen Methylierung von unterschiedlichen Kombinationen der hier offenbarten Gen-Abschnitte prädiktiv für das Vorhandensein und das Stadium eines Tumors, insbesondere eines Prostata-Tumors, sind. Dabei können Gen-Abschnitte hypermethyliert und/oder auch hypomethyliert sein. Für die Diagnose werden folglich unterschiedliche Gen-Abschnitte auf deren differentielle Methylierung untersucht. Die erhaltenen Ergebnisse zur Methylierung bzw. zur differentiellen Methylierung der einzelnen Gen-Abschnitte ist prädiktiv für einen Tumor oder das Stadium eines Tumors.

Unterschiedliche Gen-Abschnitte im Sinne der Erfindung bedeutet, dass mehrere oder eine Vielzahl der hier offenbarten Gen-Abschnitte mit differentieller Methylierung herangezogen werden und die so gewonnene Methylierungssignatur zur Diagnose herangezogen wird. Es können beispielsweise 2 bis 4, 5, 10, 15, 20 oder mehr Gen-Abschnitte berücksichtigt werden. Im Gegensatz zu dem Verfahren der WO 2010/084154 A2 werden im erfindungsgemäßen Verfahren auch Hypermethylierungen von Gen-Abschnitten berücksichtigt. Erfindungsgemäß werden die Hypomethylierung und/oder die Hypermethylierung herangezogen.

In dem erfindungsgemäßen Verfahren wird vorzugsweise die differentielle Methylierung der hier offenbarten Gen-Abschnitte untersucht und zur Diagnose verwendet. Jeder individuelle Tumor weist eine spezifische DNA-Methylierungssignatur auf. Dazu gehören verschiedene Kombinationen aus hyper- und hypomethylierten CpG reichen Genomsequenzen. Das erfindungsgemäße Verfahren ermöglicht den Nachweis dieser Kombinationen auch vor dem Hintergrund der bekannten genetischen Heterogenität der zellulären Genome des Tumorgewebes. Somit kann der Tumor indirekt nachgewiesen werden.

Erfindungsgemäß wird also eine Probe genomischer DNA eingesetzt. Die Probe genomischer DNA kann grundsätzlich aus unterschiedlichen Quellen stammen, beispielsweise durch Aufreinigung aus Zellproben erhalten werden. Geeignete Zellproben könnten beispielsweise im Rahmen einer Biopsie gewonnen werden; es kann sich jedoch auch um Blutproben, Speichelproben, Urinproben oder ähnliches handeln.

In einem Schritt erfolgt nun die quantitative Bestimmung der Gegenwart des DNA-Abschnitts in der Probe. Zusätzlich erfolgt die quantitative Bestimmung des Vorliegens von zumindest einem differentiell methylierten C eines CpG-Dinukleotids innerhalb des DNA-Abschnitts.

Unter "quantitativer Bestimmung" ist eine Detektion des Vorhandenseins zu verstehen. Dabei soll nicht eine bloße qualitative Detektion erfolgen, d.h. die Frage beantwortet werden, ob ein DNA-Abschnitt vorhanden ist, sondern dieses Vorhandensein auch quantifiziert werden (zum Beispiel durch Mengenangaben, Anzahlen von Kopien und dergleichen). Dem Fachmann sind verschiedene Verfahren bekannt, um eine solche quantitative Bestimmung durchzuführen. In einer bevorzugten Ausführungsform erfolgt diese quantitative Bestimmung über eine Amplifikation mit anschließender Messung der Menge des erzeugten Amplifikats. In einer bevorzugteren Ausführungsform ist die Amplifikation eine PCR. In einer weiteren noch bevorzugteren Ausführungsform erfolgt die quantitative Bestimmung mittels Real Time PCR durch Bestimmung des CT-Werts.

In einer weiteren Ausführungsform erfolgt die quantitative Bestimmung über die Hybridisierung einer markierten Sonde (z.B. Nukleinsäuresonde) mit anschließender Bestimmung der Höhe des durch die Markierung (direkt oder indirekt) erzeugten Signals. In einer weiteren Ausführungsform wird eine in situ Hybridisierung (z.B. FISH) durchgeführt mit anschließender Bestimmung der Höhe des durch die Markierung erzeugten Signals. Weitere Verfahren für solche quantitative Bestimmungen sind die Detektion mit 5-methyl-Cytosin-spezifischen Antikörpern, oder der indirekte Nachweis von an methylierte DNA bindenden Faktoren mittels spezifischer Antikörper.

Unter "differentiell methyliertes C eines CpG-Dinukleotids" wird vorliegend der in verschiedenen möglichen Ausprägungsformen vorliegende Methylierungszustand des betreffenden Cytosins verstanden. Dieser kann entweder methyliert sein, d.h. dieses Cytosin liegt als 5mC vor, oder er kann unmethyliert (oder demethyliert) sein, d.h. das betrachtete Cytosin weist keine 5-Methylgruppe auf. Differentiell methyliert bezieht sich also nur auf die Möglichkeit, methyliert oder unmethyliert vorliegen zu können.

Die differentielle Methylierung ergibt sich aus der Betrachtung einer Vielzahl übereinstimmender DNA-Abschnitte in der Probe. Beispielsweise könnte die Probe genomischer DNA aus einer Probe mit 10.000 Zellen stammen. Während jedes einzelne Cytosin nur eine eindeutige Methylierung oder Nicht-Methylierung haben kann, kann sich der Grad der differentiellen Methylierung der Gesamtprobe unterscheiden, z.B. könnte ein Cytosin im DNA-Abschnitt in der Probe zur Hälfte methyliert und zur Hälfte nicht-methyliert vorliegen in einer anderen Probe könnte der gleiche DNA-Abschnitt zu 90% methyliert vorliegen.

Ein DNA-Abschnitt im Sinne der Erfindung ist kein Transposon. Er ist ein Abschnitt aus dem Genom, der nur einmal oder mehrfach, aber nicht repetitiv vorkommt. Nicht repetitiv bedeutet, dass der DNA-Abschnitt beispielsweise bis zu dreimal, zehnmal höchstens aber bis zu 1.500 Mal im Genom vorkommt. Ein DNA-Abschnitt muss daher eine gewisse Länge aufweisen, um genügend Sequenzspezifität aufzuweisen, um ihn von anderen Genabschnitten zu unterscheiden. Auf der anderen Seite darf sich der Genabschnitt nicht auf verschiedenen Chromosomen oder verschiedenen Loci eines Chromosoms wiederholen. Vorzugsweise hat ein DNA-Abschnitt eine Länge von mindestens 100 Basenpaaren, bevorzugter mindestens 1.000 oder 10.000 Basenpaare; besonders bevorzugt kürzer als 2.000.000 oder 1.000.000 Basenpaare oder 100.000 Basenpaare.

Ein Genlocus ist die physische Position eines Gens im Genom. Besteht das Genom aus mehreren Chromosomen, ist der Genlocus der Ort auf dem Chromosom, in dem sich das Gen befindet. Verschiedene Ausprägungen oder Varianten dieses Gens werden als Allele bezeichnet, die sich alle an der gleichen Stelle auf dem Chromosom, nämlich dem Genlocus befinden.

Typischerweise liegt die Länge eines erfindungsgemäßen DNA-Abschnitts bei nicht mehr als 2,5 Mio. Basenpaaren, bevorzugt bei ≤ 1 Mio. Basenpaaren oder ≤ 500.000 Basenpaaren.

Das Genom umfasst Gene und intergenische Bereiche. "Gene" umfasst Bereiche, die durch Transkription in einzelsträngige mRNA überführt werden und zusätzliche DNA-Abschnitte, die regulatorische Funktion haben, beispielsweise Promotoren und Enhancer. Die durch Transkription abgelesenen Bereiche umfassen wiederum Exons und Introns, wobei die Introns während der Prozessierung entfernt werden. Grundsätzlich können auch Exons und Introns weitere regulatorische Elemente umfassen.

Der erfindungsgemäß verwendete Begriff "DNA-Abschnitt" umfasst somit sowohl intergenische Bereiche als auch Gene und bei Genen sowohl regulatorische Bereiche als auch Introns und Exons.

Erfindungsgemäß entscheidend ist nur, dass der DNA-Abschnitt nur an einem Genlocus vorkommt, um eine spezifische Aussage über den Methylierungsgrad dieses DNA-Abschnitts treffen zu können.

Kommt ein DNA-Abschnitt innerhalb des Genoms an verschiedenen Stellen vor, ergeben sich Methylierungsgrade, die nicht für den DNA-Abschnitt spezifisch sind, sondern gemittelte Werte darstellen, deren Aussagekraft stark vermindert ist.

So könnte beispielsweise ein DNA-Abschnitt, der im Genom zweimal vorkommt in einem der Bereiche stärker und im anderen schwächer methyliert sein. Auf Grund der Bildung des Mittelwertes würde der sich daraus ergebende Methylierungsgrad unverändert erscheinen und hätte daher keine Relevanz.

Beispiele für erfindungsgemäß verwendete DNA-Abschnitte sind insbesondere Housekeeping-Gene, zelltypspezifisch exprimierte Gene, DNA-Abschnitte, die für eine micro-RNA kodieren, DNA-Abschnitte, die für Nicht-Protein kodierende RNA kodieren sowie nicht-kodierende DNA.

Beispiele für bevorzugte DNA-Abschnitte, an denen die Methylierung durch das erfindungsgemäße Verfahren untersucht werden kann, sind beispielsweise die Gene

RASSF1, GSTP1, ZIC4, CD44, CDKN1A, ESR1, PLAU, RARB, SFN, TNFRSF6, TSPY, ARHI, bcl-2, BRCA1, CDKN2C, GADD45A, MTAP, PGR, SLC26A4, SPARC, SYK, TJP2, UCHL1, WIT-1, PAK6, RAD50, TLX3, PIR51, MAP2K5, INSR, FBN1, SEPT9 und GG2-1.

Es ist das Verdienst der Erfindung, weitere Genorte aufgefunden zu haben, an denen differentielle Methylierungen vorhanden sein können. Die differentielle Methylierung an diesen Gen-Abschnitten wurde in Prostata-Karzinompatienten aufgefunden. Die in den nachfolgenden Listen 19, 20, 2 1, 22, 23, 24, 25, 26, 27, 28, 29, 30, 31, 32, 33, 34, 35, 36, 37 und 38 genannten Gen-Abschnitte, insbesondere die Gen-Abschnitte aus den Listen der Schnittmengen der aufgelisteten Gen-Abschnitte sowie Kombinationen von Gen-Abschnitten dieser Listen eignen sich somit für die medizinische Diagnose. Diese Diagnose ist eine in vitro Diagnose.

Für das Screening nach den zuvor aufgelisteten Gen-Abschnitten wurde die extrahierten DNA-Proben einer Immunopräzipitation unterworfen unter Verwendung eines Antikörpers mit Bindungspezifität für Methylcytosin. Es erfolgte eine Amplifikation der präzipitierten, methylierten DNA-Fraktion, deren Farbstoffmarkierung und die Hybridisierung auf NIMBLEGEN DNA Methylierungsarrays der Firma Roche mit allen bekannten CpG reichen DNA-Abschnitten des humanen Genoms (http://www.nimblegen.com/products /-nimbledesign/index.htmlNimbleDesign Software). Die erhaltenen Daten wurden mit dem Axon 4000B Microarray Scanner ausgelesen und die Weiterverarbeitung der Rohdaten erfolgte durch den Bioinformatiker des Max Plank Instituts Münster, Dr. Marcos Arauzo Bravo, mittels eigens entwickelten Funktionen von Matlab (http://www.mathworks.de/products/matlab/). Das Screeening Verfahren ist beschrieben in Parasitol Res. 2013 Nov;112(11):3757-70. 2013 Aug 16. Genome-wide screening identifies Plasmodium chabaudi-induced modifications of DNA methylation status of Tlr1 and Tlr6 gene promoters in liver, but not spleen, of female C57BL/6 mice. Al-Quraishy S, Dkhil MA, Abdel-Baki AA, Delic D, Santourlidis S, Wunderlich F.

Die Untersuchungen wurden mit der bekannten Methylierungs-Array-Technologie vorgenommen, die beispielsweise in der oben angegebenen Literaturstelle beschrieben ist.

### Prostata - Hypermethylierte Biomarker.

Die nachfolgend aufgelisteten Gen-Abschnitte umfassen hypermethylierte Gen-Abschnitte bei Prostatatumor-Patienten. Deren Methylierungsgrad kann als Tumormarker für Prostatatumore herangezogen werden.

### Liste 19 (Kohorte 1 - Prostataektomie):

FAM50B-Ups, XPO5-Cor, PITPNM1-Ups, LOC100419707-Cor, NTAN1-Cor, RPL36AP39-Ups, UBE2Z-Ups, TMEM80-Cor, MIR324-Ups, NR1D1-Ups, ZWINT-Cor, SLX4-Cor, HMGN2P28-Ups, MIR5696-Cor, HMGN2P28-Cor, RPS27P20-Cor, MPP7-Ups, PCBP3-Cor, MYEF2-Ups, TMEM254-Ups, ILVBL-Ups, VWC2L-Ups, CFHR5-Cor, ZSCAN1-Cor, RPL3P10-Ups, CEACAM1-Ups, MYEF2-Cor, TMSB4XP2-Cor, SCN8A-Ups, PPDPF-Cor, LOC100421695-Cor, TRNAQ15-Cor, TCTN2-Cor, PKN1-Ups, BRE-AS1-Cor, RAD21L1-Cor, JOSD2-Ups, ZNF586-Ups, OXLD1-Cor, TP53-Cor, MIR2115-Ups, EIF4E2-Cor, IGF1R-Cor, PRKRIP1-Cor, C10orf47-Cor, PCBP1-Cor, NDUFAB1-Ups, POU5F1P6-Cor, RPL22P14-Cor, KIR2DP1-Cor, MAGEB1-Ups, GTF2H3-Cor, IFNA17-Ups, TRIM62-Ups, MAGEB1-Cor, CTSW-Ups, LOC100130632-Ups, LOC100420668-Cor, ACAP1-Cor, ZNF229-Cor, FAR1-Cor, MIRLET7F2-Cor, KAT6B-Cor, LOC100379290-Ups, LOC100507165-Cor, TBX3-Ups, MIR4692-Ups, C3-Cor, MIR4324-Cor, RHOT1-Cor, DDOST-Ups, MIR500B-Ups, PLIN2-Cor, RPL7AP64-Cor, GSG2-Ups, RPL29P2-Ups, HIGD1AP12-Cor, PDGFA-Cor, MST1-Cor, ITGAD-Ups, DAK-Cor, FAM66E-Cor, WDR74-Ups, SLC22A14-Cor, HMGB3-Ups, FAM90A24P-Cor, LOC283335-Cor, MIR338-Cor, MIR548A2-Cor, ADORA2BP1-Cor, IFFO2-Cor, CALD1-Ups, POU5F1P6-Ups, ARHGEF1-Cor, CNFN-Ups, MAST1-Ups, SLC22A13-Ups, SCDP1-Ups, OR6C2-Ups, HOXB-AS1-Cor, PPIGP1-Ups, RPS12P26-Cor, RMI2-Cor, CDH13-Cor, TIGD1-Cor.

### Liste 20 (Kohorte 2 - Biopsie):

SRY-Cor, LOC100131262-Cor, BBC3-Cor, RNASEH2CP1-Cor, GLUL-Ups, ABTB1-Ups, PMS2-Cor, LOC100506710-Ups, WFS1-Ups, NTAN1-Cor, TTLL3-Cor, ITGAD-Ups, FAM66E-Cor, LOC100418770-Ups, FLJ39639-Cor, FAM90A24P-Ups, RPL36AP39-Ups, LOC100419707-Cor, TIGD1-Cor, LRRC14B-Cor, TMEM254-Ups, KIAA1609-Ups, MKNK1-AS1-Cor, TRNAC-GCA-Cor, ZNF775-Ups, MST1-Cor, GPX1-Ups, PPIGP1-Ups, KIR2DP1-Cor, HMGB3-Ups, SCAF1-Cor, LOC100419847-Ups, NOS2P2-Cor, VWC2L-Ups, RMI2-Cor, TEX38-Cor, TYMS-Cor, PSG10P-Cor, ADAM5-Ups, RAD21L1-Cor, LINC00675-Ups, C12orf60-Cor, IFNA17-Ups, ATG16L2-Ups, ATP6V1G3-Cor, TSPAN13-Cor, MIR5696-Cor, HOXB-AS1-Cor, UBIAD1-Cor, DAK-Cor, MPP7-Ups, JDP2-Ups, CNFN-Ups, LOC100506869-Cor, TMSB4XP2-Cor, PPDPF-Cor, LOC100132058-Cor, PCGF6-Ups, FAM50B-Ups, OR6C2-Ups, NRON-Ups, KCNG4-Ups, TPRG1-Cor, LZTFL1-Cor, FSCN1-Cor, ACAD8-Cor, LOC100130566-Cor, PSG8-Cor, MIR4457-Cor, MIR4457-Ups, WDR17-Ups, PLA2G1B-Cor, TRNAW2-Cor, LOC100508751-Ups, LDLRAP1-Cor, TAS2R64P-Cor, RHBDD1-Cor, SH3BP2-Cor, ATP6V0A2-Ups, CACNA1E-Cor, ASGR2-Cor, SCN8A-Ups, TCEB2-Cor, LOC101060020-Cor, MIR4692-Ups, OR51F5P-Ups, JOSD2-Ups, MIR4518-Ups, HAUS6-Cor, PIK3CG-Ups, RPP25L-Cor, MIR338-Cor, ARF4-Cor, RPL5P34-Cor, RPS2P48-Cor, HMGN2P28-Cor, POU5F1P6-Ups, TNFAIP8L1-Ups, LOC100419932-Ups, CFHR5-Cor, CALD1-Ups, C10orf47-Cor, HIGD1AP12-Cor, MIR4302-Ups, LOC724105-Ups, LMLN-Cor, TRNAQ15-Cor, DLK2-Ups, TIGIT-Cor, TUSC5-Cor, PPIAP29-Cor, RBM22P11-Ups, LOC100131262-Ups, ZSCAN23-Cor, ZNF614-Ups, TEAD2-Cor, FLJ46026-Ups, CGB5-Cor, LOC100505685-Cor, PROK2-Ups, MYEF2-Ups, MAST1-Ups, APLP2-Ups, LOC729835-Ups, LOC646956-Ups, HYDIN-Cor, LOC100887074-Ups, RPS29P27-Ups, TRNAC14-Cor, FAM159B-Cor, OR5E1P-Cor, LOC100129248-Cor, NAV2-AS5-Ups, RPS27P20-Cor, LOC645136-Cor, ASS1P13-Ups, GMNN-Cor, ADAM5-Cor, GAMT-Cor, ZNF419-Cor, NKX2-6-Cor, ZSCAN1-Cor, MIR324-Ups, ZNF428-Ups, RPS24P10-Ups, SEPT3-Cor, RPS15AP36-Cor, RGS12-Cor, ZNF233-Ups, LOC100499399-Cor, TCTN2-Cor, CYTH1-Ups, ZNF229-Ups, WHSC2-Cor, LIAS-Cor, DDB1-Ups, RPS12P26-Ups, LOC100506405-Cor, LOC728228-Cor, SCDP1-Ups, PITPNM1-Ups, HMGN2P28-Ups, RPL7AP64-Cor, PGF-Cor, KCTD11-Cor, LOC731932-Ups, LOC100533627-Ups, TRIM62-Ups, RAB1C-Cor, OR2I1P-Ups, RPS4XP12-Cor, HAND2-Cor, LOC100129566-Cor, LOC100631378-Cor, C2orf65-Ups, PLA2G1B-Ups, LRRC3C-Cor, RPL9P29-Cor, FAM86GP-Ups, TRNAG20-Ups, TRIT1-Ups, SPTAN1-Cor, NTN1-Ups, PAPOLB-Ups, MAN1C1-Ups, TRNAD9-Cor, ARPC5-Cor, KLRC1-Ups, HEATR1-Cor, CCDC48-Ups, LOC727964-Ups, RAPGEF4-Cor, ADAM7-Cor, MIR4667-Ups, PCMTD1P2-Ups, SLC22A12-Ups, CYP46A4P-Cor, ANKRD52-Cor, SNORD43-Cor, KEAP1-Ups, PUS1-Ups, PTPMT1-Cor, SPDYE3-Cor, DDB1-Cor, MTND5P20-Ups, ZNF548-Ups, HIST1H4F-Cor, RPL26P7-Ups, LOC729121-Cor, LOC100507165-Cor, SNORA39-Cor, NLRP8-Cor, IFNWP15-Cor, NDUFB8P2-Cor, ZNF586-Ups, KIF17-Cor, OR7E46P-Ups, RNF123-Cor, ARHGEF1-Cor, MTRNR2L10-Ups, LOC284933-Cor, MIR758-Cor, LOC100421525-Ups, UBE2Z-Cor, LOC731932-Cor, LOC645954-Ups, TRNAA2-Cor, CASC3-Cor, APLP2-Cor, RPL31P47-Cor, FLJ13224-Ups, RPL31P61-Ups, ADORA2BP1-Cor, PPIE-Ups, RPL31P61-Cor, ANKRD52-Ups, GOLGA2P4-Cor, ZBTB5-Cor, TTC34-Ups, RAP1BL-Ups, RBPJ-Ups, LOC100507468-Cor, CTXN2-Cor, RNF167-Cor, THAP5P1-Ups, MXD1-Ups, MIR639-Ups, MATK-Cor, MIR548O-Ups, MYL12B-Cor, LOC100131395-Ups, LOC283335-Cor, MIR711-Ups, CCDC64B-Ups, SLC38A10-Ups, CTSW-Ups, ZNF234-Cor, MAGEB1-Cor, FTL-Cor, TMEM216-Cor, FBLN2-Ups, SHANK2-AS1-Cor, PDE8B-Cor, KRT23-Ups, MIR153-2-Cor, WHSC2-Ups, TMIE-Cor, C17orfl07-Cor, RPL3P10-Ups, TAF12-Cor, PLIN2-Cor, TMCO6-Ups, MYEF2-Cor, AOC3-Ups, LENG1-Ups, FKBP10-Cor, LINC00656-Cor, SEMA5B-Cor, FAM90A24P-Cor, IRF3-Cor, GUCA1B-Ups, RNU12-2P-Ups, MIR518B-Ups, MAGEB1-Ups, LOC100506710-Cor, SH2D3C-Ups, PRRT1-Ups, GNPAT-Cor.

### Liste 21 (Kohorte 3 - Prostataektomie Tübingen):

RNASEH2CP1-Cor, HMGN2P28-Cor, PFDN4-Cor, MIR611-Cor, COX6B2-Cor, HMGN2P28-Ups, TAC3-Ups, XRCC6BP1-Cor, RAD51D-Ups, FAM90A24P-Ups, LAIR2-Cor, HOXB-AS1-Cor, C6-Ups, RPL13P7-Ups, KRT23-Ups, LOC204800-Cor, LOC100128136-Cor, POU5F1P6-Ups, FAM66E-Cor, LOC100129554-Ups, LOC100128048-Cor, PLCD3-Ups, TRBV24-1-Ups, FRMPD2-Cor, KIN-Cor, RPL23AP75-Cor, CHCHD2P7-Ups, RPS2P25-Ups, LOC100420334-Ups, ARL14EP-Ups, RSL24D1P8-Ups, OR4A42P-Cor, SEC63-Cor, MIRLET7F2-Cor, TRNAR5-Cor, LOC100996515-Ups, LOC100506343-Ups, NTAN1-Cor, GNA11-Ups, ARHGEF39-Cor, NAV2-AS5-Ups, MMD2-Ups, CYP3A4-Ups, SHANK2-AS1-Cor, MAGEB1-Ups, RAB3B-Ups, LOC100505702-Ups, MTND5P20-Cor, LETM1P3-Ups, LOC100421695-Cor, HIST1H2AE-Cor, TSGA10IP-Ups, MLF2-Ups, RBBP9-Cor, NUP50-Cor, HTR2C-Cor, RPL39P25-Cor, LOC100421361-Cor, RPS15AP36-Cor, FAM227A-Cor, MIR194-2-Cor, ZBTB47-Ups, KLK9-Ups, SNORD33-Cor, NTMT1-Cor, RPS12P14-Cor, LOC101060199-Cor, ZNF491-Cor, BRE-AS1-Cor, SLC35B4-Ups, LRRC37A4P-Ups, MPP7-Ups, ASIC2-Cor, KIF17-Ups, ZNF599-Ups, CCDC134-Ups, IL4I1-Cor, AP3B1-Cor, CSGALNACT1-Cor, SH3TC2-Cor, OR7E62P-Cor, SPTAN1-Ups, DCTN2-Ups, FAM50B-Ups, CIT-Cor, RPL36AP39-Ups, TCTN2-Cor, LOC100505995-Ups, CAPN6-Ups, DUSP28-Ups, OR51AB1P-Ups, TMUB2-Cor, KLRC1-Ups, LOC100128136-Ups, TPTE2P3-Ups, SHANK2-AS1-Ups, SMCR7L-Ups, LOC338817-Cor, AK3P3-Ups, SEPT3-Cor, UBE2WP1-Cor, RMI2-Cor, MIR874-Cor, LOC126860-Cor.

### Liste 22 (Kohorte 4 -Plasma-Tübingen):

SPIB-Ups, MIR379-Cor, TFCP2L1-Ups, RNU5E-1-Cor, LOC100130932-Ups, RAD51D-Ups, DBNL-Ups, LAP3P2-Ups, ITGB3-Cor, PPP1R9B-Cor, HMGN2P28-Cor, MIR758-Cor, HMGN2P28-Ups, RPL9P33-Cor, ANKRD11-Ups, ADCK2-Cor, ZNF439-Cor, RPL5P34-Cor, EEF2K-Cor, KLHDC7A-Ups, SRY-Ups, NADSYN1-Ups, TRBV29OR9-2-Cor, VN2R11P-Ups, ZNF524-Ups, LOC100128288-Cor, CXorf51A-Ups, PPIE-Cor, USP35-Cor, XAGE-4-Cor, TIMM17B-Ups, CD320-Ups, NDUFV1-Ups, LOC101059913-Ups, LOC101060016-Cor, LOC285972-Cor, QSER1-Ups, SLC2A3-Ups, HMGN2P9-Cor, XPO5-Cor, LOC729835-Cor, LOC100996515-Ups, LIAS-Cor, LOC728228-Cor, ZBTB20-AS1-Cor, RPL7AP64-Cor, LRRC23-Cor, LOC101059922-Cor, RPL23AP5-Ups, IFRD2-Cor, AMACR-Cor, LOC338817-Cor, LOC728815-Cor, ART2P-Cor, HMOX1-Cor, LOC100131510-Cor, VTRNA1-1-Ups, ALB-Ups, MTND1P30-Cor, ZNF788-Ups, GIMAP8-Ups, FAM159B-Cor, FOXRED1-Cor, LOC401864-Ups, DDB1-Cor, RPL7P5-Ups, MIR299-Cor, GAA-Ups, CXorf51A-Cor, LOC724105-Cor, REP15-Cor, RPS12P29-Ups, PCMTD1P2-Ups, PDZRN3-Ups, MIR630-Ups, HNRNPA3P4-Cor, LOC730974-Cor, TRNAA46P-Ups, RPS3AP43-Cor, SRY-Cor, SLC35E2-Ups, LOC101060016-Ups, SNORA60-Cor, RPL7AP64-Ups, ALOX15B-Cor, LINC00251-Ups, KIR2DS4-Ups, LGALS14-Cor, PCGF6-Cor, TMIGD2-Cor, FAM211A-Cor, SHANK1-Ups, C15orf48-Ups, MYT1L-Ups, ITIH2-Cor, LOC728228-Ups, PPP1R9B-Ups, PCMTD1P2-Cor, MIR3148-Cor, ATPAF1-Cor, ZFP36-Cor, LOC400752-Ups, STAT1-Cor, NTMT1-Cor, EIF2AP1-Cor, RPL3P10-Ups, COX5BP8-Cor, RBBP9-Cor, SH3YL1-Cor, NAT8L-Ups, SNRPFP4-Cor, MIR4300-Cor, RPS27P20-Cor, SLC28A2-Ups, HSPD1P5-Ups, LOC100996878-Cor, BTN2A3P-Ups, EPN3-Cor, FAM104B-Cor, ALKBH7-Cor, HMGN2P2-Ups, ZNF550-Cor, RPL13P7-Ups, TMEM75-Ups, FAM87B-Ups, TSHZ2-Cor, ASCL2-Ups, LOC100129554-Cor, PTPLAD1-Cor, SNORA5A-Cor, RSL24D1-Cor, LOC100996515-Cor, PLN-Cor, MTRNR2L10-Ups, RPS28P3-Ups, OR13J1-Ups, EIF4A1P3-Ups, HOXB-AS1-Cor, MIR4653-Ups, MYH9-Cor, LOC100996904-Ups, FBLN2-Ups, MFI2-AS1-Ups, RNU7-82P-Ups, SLC12A1-Ups, LOC728815-Ups, PCDHAC2-Ups, ZNF229-Cor, FAM66E-Ups, SERP2-Ups, OR52P1P-Ups, PRRG1-Cor, LOC100506844-Ups, CCDC105-Ups, LOC100422633-Cor, C8orf48-Ups, RNASEH2CP1-Ups, LOC100271722-Cor, MKNK1-AS1-Cor, SH3GL1P3-Cor, LOC729327-Cor, OR51C4P-Ups, FAM86B2-Ups, RPL17P22-Ups, UNC93B2-Ups, RPS3AP19-Cor, LOC100506314-Cor, LZTFL1-Ups, DCTN6-Ups, RPL10P3-Cor, C1orf122-Ups, MYO1A-Cor, PRRT1-Ups, MIR4439-Cor, WFIKKN1-Cor, ADPGK-Ups, TRNAM6-Cor, TESPA1-Cor, MLF2-Cor, BRI3BP-Cor, LPPR3-Cor, RPS10P21-Ups, SLBP-Cor, JOSD2-Cor, CIDEC-Ups, SLC6A12-Cor, LOC100419847-Ups, CIT-Cor, LAIR1-Ups, XPNPEP1-Ups, G6PC3-Ups, MIR5003-Ups, PDXK-Ups, LOC389120-Ups, PRDX3P2-Ups, LOC401679-Cor, RIMKLA-Cor, ZNF541-Ups, MIR5703-Ups, LOC724105-Ups, EFHC1-Ups, ADAD2-Ups, SEC23B-Cor, DCAF10-Cor, NR1D1-Ups, ECSCR-Ups, RNASEH2CP1-Cor, PROK2-Ups, LOC100289388-Cor, NRIP3-Ups, ST6GAL1-Ups, TMSB4XP5-Ups, CUL1-Ups, LOC646801-Cor, OR7E1P-Ups, GNLY-Ups, ASCL2-Cor, HYDIN-Cor, OR52A1-Cor, HNRNPA3P4-Ups, LOC100134391-Ups, OR3A3-Cor, PPAP2C-Cor, LOC100310848-Cor, HIATL2-Ups, VWC2L-Ups, NXPH3-Cor, MAS1-Cor, ZNF491-Cor, CWF19L1-Cor, LOC728549-Cor, LOC100131536-Ups, C9orf72-Ups, EPN3-Ups, RNF2-Ups, LOC100420450-Cor, MIR584-Cor, LOC100506710-Ups, TMPOP1-Ups, LOC645749-Cor, TRIM6-Ups, LOC101060199-Cor, MIR3117-Cor, WRNIP1-Ups, LOC400752-Cor, WNT3-Ups, RPS3AP43-Ups, RPS27P3-Ups, DSG1-Cor, LOC100128373-Cor, TBX3-Cor, APIP-Ups, PSG8-Cor, PSMA4-Cor, ZNF8-Ups, PGK1-Cor, BRD4-Cor, MIR376A2-Cor, IL29-Cor, LOC100420423-Cor, LOC100996904-Cor, LOC390099-Ups, TAS2R68P-Cor, LOC285972-Ups, SRM-Ups, TPI1-Ups, LOC100420404-Ups, ROM1-Ups, MFI2-AS1-Cor, ZCCHC5-Cor, IFNWP15-Cor, SLC26A10-Ups, MTND5P20-Cor, BAIAP2-AS1-Cor, THBS4-Ups, ARMCX6-Ups, CYP3A4-Ups, LOC101060826-Cor, ECI2-Cor, COL5A3-Cor, LOC100506877-Ups, WDR81-Cor, LOC100129940-Cor, TRNAP24P-Cor, E4F1-Ups, SELV-Ups, COG1-Ups, LOC100509638-Cor, PTP4A1P2-Ups, LOC100421341-Ups, ZNF439-Ups, LOC100419684-Cor, ZNF502-Cor, KIF1A-Ups,FAM150B-Cor, MIR638-Cor, RHOT1P1-, or, KCNK4-Ups, ILF3-AS1-Cor, ADAD2-Cor, LOC100506928-Ups, IRF2BP1-Cor, LOC100420057-Ups, MIR199A1-Cor, IGF2BP1-Ups, NLRP1-Ups, FAM57A-Ups, PTX4-Cor, NLE1-Ups, LSM12-Ups, PRRG1-Ups, MIR4794-Ups, OR8A1-Cor, LOC100128048-Cor, MIR4261-Cor, BRI3BP-Ups, POLR2LP-Cor, PRR25-Cor, RNF216-IT1-Cor, KRTAP26-1-Ups, RPL15P15-Cor, PAX5-Ups, RALB-Cor, PDCL3P1-Ups, SLC27A1-Ups, MRPL16-Cor, MAGEA11-Ups, TRNAA46P-Cor, MC1R-Ups, ZNF749-Ups, RHOT1-Ups, LOC100506730-Ups, TPTE2P3-Ups, HIST1H4D-Ups, LINC00162-Ups, LOC100130618-Cor, LOC100293748-Ups, CLDND2-Ups, EML6-Ups, TCTN3-Cor, PSMB8-Cor, LOC284379-Ups, RSPO2-Ups, RANGAP1-Ups, LOC100418770-Cor, LOC101059948-Ups, LOC402096-Ups, DND1P2-Ups, C1orf222-Ups, SGK223-Cor, IQCF5-Ups, USP24-Cor, TRNAV20-Ups, DAK-Cor, PPP5D1-Cor, RHBDD2-Cor, NFKBID-Cor, TRNAS7-Ups, CHMP2A-Ups, LOC100533638-Cor, SMPDL3B-Cor, TRAPPC2P1-Cor, OR13C7P-Cor, TUBG2-Ups, FAM86B2-Cor, LAIR1-Cor, NDUFB3P5-Cor, IREB2-Cor, OR51M1-Cor, PTMS-Cor, GSPT1-Cor, LOC100419684-Ups, ZRANB3-Cor, ZNF884P-Ups, OR51M1-Ups, CSTA-Ups, ABCA17P-Cor, UQCRHP1-Cor, KCNK16-Cor, EIF4E3-Cor, SLC16A7-Ups, LOC100420074-Ups, LOC100134362-Cor, C10orf47-Ups, FLYWCH1-Cor, LOC101060817-Cor, HCAR3-Cor, OR56B3P-Cor, LOC100419827-Cor, ODC1-Ups, CRNKL1-Ups, MIR516B2-Cor, SPDYE3-Cor, LOC100996630-Cor, LOC100509196-Ups, LOC100505702-Cor, CHCHD2P4-Ups, CRYGEP-Ups, SPAG6-Ups, OR2V2-Ups, USP10-Cor, TMEM194A-Ups, MLLT1-Ups, RPUSD4-Cor, TPI1P4-Ups, OR10AE3P-Cor, YRDC-Cor, LOC100289308-Ups, NAV2-AS4-Cor, ABTB1-Cor, C21orf90-Ups, TRNAA44P-Cor, HDGFP1-Ups, EML3-Cor, UBE2G2-Cor, SIPA1-Cor, OR5A2-Ups, GLUL-Ups, LOC100132058-Cor, LOC100293704-Cor, JUND-Ups, HFE-Ups, LOC100505754-Cor, H1FX-Ups, LOC100653200-Cor, SPATA32-Ups, LOC440489-Ups, LOC100505754-Ups, MTND5P20-Ups, TRMT6-Cor, ITGB7-Cor, ARHGEF39-Cor, AIP-Ups, SLC25A6P1-Ups, SYF2-Ups, PPFIA3-Cor, IFNA7-Ups, RPL29P9-Cor, EPN1-Ups, DAK-Ups, LOC100420255-Ups, LOC100124402-Cor, RPL31P12-Ups, LHFPL3-AS1-Cor, RANGRF-Cor, PITPNM1-Cor, NAGLU-Cor, LOC285095-Ups, TTC9B-Ups, REPIN1-Cor, CCDC171-Cor, LOC100419068-Ups, LOC100506861-Cor, IFNA12P-Cor, SPDYE3-Ups, SNRPGP3-Cor, ART1-Ups, SLC9A2-Cor, TM4SF20-Cor, NLGN2-Cor, DAPP1-Cor, LOC100506405-Cor, C21orf90-Cor, CALCOCO1-Ups, KIAA1456-Cor, OSTCP8-Cor, LPP-AS2-Cor, LOC401777-Cor, HSBP1-Ups, C6orf62-Ups, SEPT3-Cor, LOC100420678-Cor, TAS2R67P-Ups, LOC100419848-Ups, SLC9A8-Ups, ADAM7-Ups, VN1R106P-Cor, LOC100996307-Ups, HIST1H1PS2-Cor, EBLN2-Ups, LOC100421074-Ups, MRFAP1L1-Ups, C3-Ups, CKAP2-Ups, SLC2A10-Ups, LOC100419839-Ups, ARF4-Cor, RPS17P10-Ups, LOC145757-Ups, MIR4270-Cor, RPS15AP26-Cor, PHB-Cor, LOC391465-Cor, RPL36AP13-Cor, SCARNA10-Cor, SEC22C-Ups, FLJ39639-Cor, RPL21P30-Ups, TRNAM15-Cor, MIR3164-Ups, RPL39P17-Ups, HLTF-AS1-Ups, ATXN7L1-Cor, NDUFA4L2-Ups, OR5AN2P-Cor, TM7SF2-Ups, LOC100130321-Cor, ARHGEF39-Ups, MYH4-Ups, ZNF132-Cor, DHX35-Cor, AK3P3-Ups, ZNF628-Ups, LOC649324-Cor, TRGVA-Cor, ZNF584-Cor, TMEM189-UBE2V1-Ups, MIR1304-Cor, CCNJP1-Cor, SEMA5B-Ups, RPL9-Cor, RPS24P10-Ups, OR1L3-Ups, GOLGA4-Cor, OR51B8P-Ups, HYDIN-Ups, PP12719-Ups, RNU7-60P-Cor, SLC25A6P1-Cor, TAF6L-Ups, LOC100288241-Cor, PCDHA7-Ups, LOC100130527-Ups, CIAPIN1P-Cor, LOC100310848-Ups, CCDC121-Cor, IL23A-Cor, RPL23AP56-Ups, DLL3-Cor, RHOT2-Cor, TSPAN16-Ups, RPL13AP16-Cor, C10orf131-Cor, TMIGD2-Ups, LOC101059948-Cor, LOC401052-Cor, SLC22A14-Ups, LOC643308-Cor, LACC1-Cor, LOC100287169-Cor, LSM14B-Cor, C20orf78-Cor, CPN1-Cor, FSD1-Ups, IFNA13-Ups, EML3-Ups, LOC100420042-Cor, SLFN11-Ups, RPL39P19-Cor, MIR3677-Cor, SMIM1-Cor, HBEGF-Cor, SNORA52-Ups, MYH9-Ups, LOC100509686-Cor, NPW-Cor, ECSCR-Cor, CD5-Ups, LOC100128770-Cor, DAZAP1-Ups, EXOC4-Cor, RPS9-Cor, RAD23A-Ups, MSL2-Ups, MIR526A1-Ups, REPS1-Cor, NDUFS7-Cor, RPEP3-Cor, MIR4288-Ups, DHCR7-Cor, C11orf42-Ups, LOC645763-Ups, LOC101059926-Cor, HBS1L-Ups, CDCA3-Cor, CBX1P5-Cor, RUNX3-Cor, HMGN2P8-Ups, EIF2AP1-Ups, LOC100421523-Ups, LOC100420910-Ups, LINC00482-Ups, C1orf109-Ups, URM1-Ups, CTCF-Ups, LOC126860-Cor, ENTPD6-Ups, SNORA70C-Cor, ACTBL2-Cor, RPL39P25-Cor, OR8G5-Ups, SNORD14E-Cor, ELAVL1-Cor, UHRF1-Ups, SUSD1-Ups, LOC101060827-Ups, TIMM44-Ups, CLCN3-Ups, PSMB7-Ups, LOC100652772-Ups, PHTF1-Ups, LOC100130169-Ups, MAFK-Cor, NUDT11-Cor, MIMT1-Cor, ITGA9-Cor, ZNF428-Cor, SERGEF-Cor, LOC100996569-Cor, FOXP1-Ups, ATP8A1-Cor, SC5DL-Ups, EN2-Ups, ZNF524-Cor, LAMB2-Ups, PALM-Cor, KIR3DL2-Cor, OR4A42P-Cor, ITGAV-Ups, MIR3616-Ups, IDO2-Cor, LOC155060-Cor, RAVER1-Cor, SLC12A1-Cor, LSM14B-Ups, A1BG-AS1-Ups, CREB5-Ups, LOC100507325-Cor, ITGAD-Ups, MIR3123-Ups, SHISA7-Ups, PGK1-Ups, OR9R1P-Ups, PRRT1-Cor, CDC25A-Ups, LOC84931-Cor, LOC100131492-Ups, MYL10-Ups, LOC100379290-Cor, NDUFB7-Ups, PGLYRP1-Cor, ZKSCAN3-Cor, ZNF323-Ups, OR6U2P-Ups, RNMTL1-Cor, DNAH11-Ups, TCEB1-Ups, PROK2-Cor, TPM4-Cor, PDZRN3-Cor, INS-IGF2-Ups, SNORA39-Cor, RPL17P22-Cor, LSM6P2-Cor, C19orf66-Cor, PP12719-Cor, AP4B1-Cor, LOC100418770-Ups, RPA3-Ups, ORAI3-Cor, ROGDI-Ups, LOC100996282-Cor, LOC100996366-Cor, LOC100132849-Cor, OR52N5-Ups, LAMB2-Cor, TSGA10IP-Ups, LOC100130618-Ups.

### Liste 23 (Schnittmenge Kohorten 1 und 2):

FAM50B-Ups, PITPNM1-Ups, LOC100419707-Cor, NTAN1-Cor, RPL36AP39-Ups, MIR324-Ups, HMGN2P28-Ups, MIR5696-Cor, HMGN2P28-Cor, RPS27P20-Cor, MPP7-Ups, MYEF2-Ups, TMEM254-Ups, VWC2L-Ups, CFHR5-Cor, ZSCAN1-Cor, RPL3P10-Ups, MYEF2-Cor, TMSB4XP2-Cor, SCN8A-Ups, PPDPF-Cor, TRNAQ15-Cor, TCTN2-Cor, RAD21L1-Cor, JOSD2-Ups, ZNF586-Ups, C10orf47-Cor, KIR2DP1-Cor, MAGEB1-Ups, IFNA17-Ups, TRIM62-Ups, MAGEB1-Cor, CTSW-Ups, LOC100507165-Cor, MIR4692-Ups, PLIN2-Cor, RPL7AP64-Cor, HIGD1AP12-Cor, MST1-Cor, ITGAD-Ups, DAK-Cor, FAM66E-Cor, HMGB3-Ups, FAM90A24P-Cor, LOC283335-Cor, MIR338-Cor, ADORA2BP1-Cor, CALD1-Ups, POU5F1P6-Ups, ARHGEF1-Cor, CNFN-Ups, MAST1-Ups, SCDP1-Ups, OR6C2-Ups, HOXB-AS1-Cor, PPIGP1-Ups, RMI2-Cor, TIGD1-Cor.

Die Gen-Abschnitte der Schnittmenge der Kohorten 1 und 2 eignen sich bevorzugt für eine frühe Diagnose eines Tumors, insbesondere eines Prostatatumors.

### Liste 24 (Schnittmenge Kohorten 1 und 3):

FAM50B-Ups, NTAN1-Cor, RPL36AP39-Ups, HMGN2P28-Ups, HMGN2P28-Cor, MPP7-Ups, LOC100421695-Cor, TCTN2-Cor, BRE-AS1-Cor, MAGEB1-Ups, MIRLET7F2-Cor, FAM66E-Cor, POU5F1P6-Ups, HOXB-AS1-Cor, RMI2-Cor.

Die Gen-Abschnitte der Schnittmenge der Kohorten 1 und 3 eignen sich bevorzugt für eine frühe Diagnose eines Prostatatumors.

### Liste 25 (Schnittmenge Kohorten 2 und 3):

RNASEH2CP1-Cor, NTAN1-Cor, FAM66E-Cor, FAM90A24P-Ups, RPL36AP39-Ups, RMI2-Cor, HOXB-AS1-Cor, MPP7-Ups, FAM50B-Ups, HMGN2P28-Cor, POU5F1P6-Ups, NAV2-AS5-Ups, SEPT3-Cor, RPS15AP36-Cor, TCTN2-Cor, HMGN2P28-Ups, KLRC1-Ups, KRT23-Ups, MAGEB1-Ups.

Die Gen-Abschnitte der Schnittmenge der Kohorten 2 und 3 eignen sich bevorzugt für eine frühe Diagnose eines Prostatatumors.

### Liste 26 (Schnittmenge Kohorten 3 und 4):

RNASEH2CP1-Cor, HMGN2P28-Cor, HMGN2P28-Ups, RAD51D-Ups, HOXB-AS1-Cor, RPL13P7-Ups, LOC100128048-Cor, OR4A42P-Cor, LOC100996515-Ups, ARHGEF39-Cor, CYP3A4-Ups, MTND5P20-Cor, TSGA10IP-Ups, RBBP9-Cor, RPL39P25-Cor, NTMT1-Cor, LOC101060199-Cor, ZNF491-Cor, CIT-Cor, TPTE2P3-Ups, LOC338817-Cor, AK3P3-Ups, SEPT3-Cor, LOC126860-Cor.

Die Gen-Abschnitte der Schnittmenge der Kohorten 3 und 4 eignen sich bevorzugt für eine frühe Diagnose eines Prostatatumors.

### Liste 27 (Schnittmenge der Kohorten 1, 2, 3):

FAM50B-Ups, NTAN1-Cor, RPL36AP39-Ups, HMGN2P28-Ups, HMGN2P28-Cor, MPP7-Ups, TCTN2-Cor, MAGEB1-Ups, FAM66E-Cor, POU5F1P6-Ups, HOXB-AS1-Cor, RMI2-Cor.

Die Gen-Abschnitte der Schnittmenge der Kohorten 1, 2 und 3 eignen sich bevorzugt für eine frühe Diagnose eines Tumors, insbesondere eines Prostatatumors.

### Liste 28 (Schnittmenge der Kohorten 1, 2, 3, 4):

HMGN2P28-Ups, HMGN2P28-Cor, HOXB-AS1-Cor.

Die Gen-Abschnitte der Schnittmenge der Kohorten 1, 2, 3 und 4 eignen sich bevorzugt für eine frühe Diagnose eines Tumors, insbesondere eines Prostatatumors.

### Prostata-HypomethylierteBiomarker

### Liste 29 (Kohorte 1 Prostatektomie-Düsseldorf):

WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, LOC401317-Cor, CRNKL1-Ups, ZHX3-Ups, RPL23AP32-Ups, MCCD1P2-Ups, DSG1-Cor, HMGN2P16-Ups, ANKRD12-Cor, PRORSD1P-Cor, SLC22A24-Ups, CD27-AS1-Cor, ZNF26-Cor, FAM131C-Ups, PPIE-Cor, NXPH4-Cor, MEN1-Ups, KDM3A-Cor, ARHGAP35-Cor, TRNAA2-Ups, EIF1AD-Ups, TPI1-Ups, ACTN3-Cor, HIST1H2BD-Cor, KCNG4-Ups, GUSBP5-Ups, SPTY2D1-Cor, LOC147670-Cor, MIR4789-Ups, KLK10-Cor, ZNF884P-Ups, YWHAB-Ups, HYDIN-Cor, PKD2L1-Ups, MIR506-Cor, PRKCG-Cor, APITD1-CORT-Ups, SNAR-D-Cor, ARF4-Cor, CYTH3-Cor, GOLGA6L4-Cor, ASCL2-Ups, ZNF132-Cor, GUCY1A3-Cor, HMOX1-Cor, PPP1R2-Ups, WBSCR17-Cor, OR52A1-Ups, PCDHAC2-Ups, LOC100506877-Ups, CXorf51A-Ups, LPAR5-Cor, ARSH-Cor, ALDH7A1P3-Cor, NAGLU-Ups, ACRBP-Cor, ZNF304-Ups, LOC100288596-Cor, MIR1972-2-Ups, TMEM33-Ups, GRIFIN-Ups, MIR548AK-Cor, SLC2A3-Ups, YBX2-Cor, LOC100419839-Ups, EPHX3-Ups, ATIC-Cor, C1R-Cor, SNORD88B-Cor, NDUFB7-Cor, HNRNPA3P4-Ups, NTMT1-Ups, BRD4-Cor, ATG2A-Cor, ARHGEF25-Ups, ZRANB3-Cor, TRNAS25-Cor, LOC100131510-Cor, CDC42EP5-Ups, TIMM8BP2-Ups, KCNK1-Cor, CHI3L2-Ups, RIMKLA-Cor, RPL12P42-Ups, PTCH2-Ups, LOC724105-Ups, LOC100130490-Cor, PCSK4-Cor, HBG2-Ups, APLP2-Ups, PCGF6-Cor, SH3BP2-Cor, DDB1-Cor, RPS29P25-Ups, A1BG-AS1-Cor, FAM90A11P-Cor, LOC100419895-Cor, RPL9P17-Ups, LOC100533638-Cor, IZUM02-Ups, HNRNPA1P19-Ups, PCBP3-Ups, HMGN2P4-Ups, TBX5-AS1-Ups, ASS1P13-Cor, LOC100419848-Ups, ZNF415-Cor, LZTS1-AS1-Cor, LOC145757-Cor, DYNC1LI1-Ups, AURKC-Ups, EPHA10-Cor, PPFIA3-Cor, TRNAV20-Ups, INSR-Cor, LARP1P1-Cor, SNORA5A-Cor, KBTBD11-Ups, ZNF187-Ups, LOC100996788-Ups, ORAI3-Ups, LOC100507530-Ups, LRRC23-Cor, VSTM2L-Ups, ACTR5-Cor, MIR4518-Cor, CCDC141-Ups, A1BG-AS1-Ups, SUMO1P1-Cor, LOC100130996-Ups, POF1B-Ups, FAM90A11P-Ups, LOC100130394-Ups, WBP1-Cor, LOC284080-Cor, ACPT-Cor, LOC100419068-Ups, RPL23AP18-Cor, ODF2L-Cor, LOC100996366-Cor, ITGAM-Cor, PRPS1P2-Ups, SEC63-Ups, GLRXP3-Cor, MIR526A2-Cor, SHANK1-Ups, APITD1-CORT-Cor, MAS1-Cor, MIR516B2-Cor, MIR376A1-Cor, LOC100130632-Cor, GAA-Ups, CIB3-Cor, CHST9-Cor, ZNF788-Ups, LOC100418940-Cor, SPHK2-Cor, ANKRD16-Cor, TRNAR12-Cor, LOC100422416-Ups, OR7E1P-Cor, EIF4E3-Cor, GPR77-Cor, TMEM254-AS1-Ups, GAPDHP37-Cor, CTXN2-Cor, LAP3P2-Ups, C16orf46-Ups, XAGE-4-Ups, ZNF135-Cor, LOC100631378-Cor, LOC100422581-Cor, RPS2P48-Cor, FAM87B-Ups, LOC644881-Cor, SNORD12B-Cor, LOC100420318-Cor, RPS29P24-Cor, OR1E1-Ups, OR6U2P-Ups, MAPKAP1-Ups, STAT1-Cor, LOC100996515-Ups, LOC100422241-Ups, TTC9B-Ups, TRNAS7-Ups, LSMD1-Ups, ADC-Ups, LOC100128398-Cor, TM7SF2-Ups, CIT-Ups, LOC100420057-Cor, MFI2-AS1-Ups, MIR5703-Cor.

### Liste 30 (Kohorte 2 Biopsie-Düsseldorf):

MMP26-Cor, TRNAV20-Ups, LOC401317-Cor, LOC390203-Ups, ZNF26-Cor, WBP11-Cor, ARPC4-Cor, TMEM33-Ups, TAF6L-Cor, NDUFB7-Cor, RILPL2-Ups, KIR2DL3-Cor, UBXN6-Ups, HMOX1-Cor, MEN1-Ups, TAF6L-Ups, LOC100507223-Cor, PROCR-Ups, OR52P1P-Ups, C11orf74-Cor, OR52B6-Ups, LOC100996630-Cor, OR52P2P-Cor, ACRBP-Cor, RPEP3-Ups, SRPR-Cor, RNU5B-4P-Cor, MIR548A2-Ups, C6orf70-Cor, OR52J3-Cor, BRD4-Cor, ASCL2-Cor, ASCL2-Ups, OR3A3-Cor, FOXRED1-Cor, MAPK8IP3-Cor, CCDC92-Cor, LOC100419839-Ups, DSG1-Cor, TRNAA2-Ups, PIM1-Cor, TRNAR3-Ups, C1R-Cor, PGP-Cor, PITPNM2-Ups, LPP-Cor, LOC729732-Cor, LOC84931-Cor, C1R-Cor, TADA3-Cor, LOC728716-Ups, PIEZO1P1-Ups, SRM-Ups, SLC26A11-Cor, RFX2-Ups, SUGP1-Cor, RPL23AP18-Cor, HMGN2P2-Ups, RPA3-Ups, FAM104B-Cor, RHBDD2-Cor, EIF1AD-Ups, ARHGAP35-Cor, MIR3972-Ups, B4GALNT4-Ups, RPS3AP9-Ups, LOC100533673-Ups, ALDH7A1P3-Cor, SERP2-Ups, LHFPL3-AS1-Ups, LINC00251-Ups, MIR1972-2-Ups, PPP1R2-Ups, SLC22A24-Ups, ACPT-Cor, ALOX15P1-Cor, MIR411-Cor, MIR4424-Ups, KDM3A-Cor, MIR194-2-Cor, SNORD88B-Cor, PHKA2-AS1-Ups, MIRLET7F2-Cor, C5orf44-Cor, DNM2-Ups, ZNF528-Ups, TRNAS22-Cor, KBTBD11-Cor, SNX15-Cor, NBLA00301-Cor, LOC100130394-Ups, CCDC114-Ups, TMPOP1-Ups, RPIAP1-Cor, LOC100130632-Ups, RPS15AP32-Ups, LOC101059913-Ups, SLC9A8-Ups, BRE-AS1-Cor, LOC100310851-Ups, ACTN3-Cor, WNT8B-Cor, LOC100506305-Cor, MIR516B2-Ups, LOC100129381-Cor, MIR4269-Cor, OR51F5P-Cor, LOC145757-Cor, RSL24D1-Cor, LOC646193-Ups, RCL1-Cor, ODF2L-Ups, RPL12P42-Ups, FUT5-Cor, TIMM8BP2-Ups, GPX1-Cor, HIST1H3F-Cor, ARGFXP2-Ups C7orf65-Cor, LOC100418848-Cor, CHMP2A-Ups, NAALAD2-Cor, NUP50-Cor, LOC100422241-Ups, HIST1H2APS4-Cor, CA8-Cor, REPIN1-Cor, VTRNA1-1-Ups, GAPDHP37-Ups, PDCD2-Ups, MRPL28-Ups, CHIAP1-Ups, SPIB-Ups, LOC285626-Cor, RNY5P6-Ups, PLEKHA8P1-Cor, C16orf46-Ups, MCCD1P2-Ups, MAU2-Cor, C9orf133-Cor, OR52J2P-Ups, SYDE1-Cor, BCL2L15-Cor, ZNF132-Cor, CRNKL1-Ups, LOC389120-Ups, LZTS1-AS1-Cor, SNORA60-Cor, CDK2AP1-Cor, ARSH-Cor, MIR518B-Cor, ASS1P13-Cor, LOC100506753-Cor, LOC100421161-Cor, PHTF1-Ups, RIMKLA-Cor, DLGAP1-AS3-Cor, LOC100419848-Ups, NDEL1-Cor, ODF2L-Cor, PPFIA3-Cor, NAGLU-Ups, ZNF304-Ups, LOC100527964-Ups, REP15-Ups, EIF4G3-Ups, HIATL2-Ups, ZNF534-Cor, FTLP13-Ups, RPS26-Cor, LOC115110-Cor, NTMT1-Ups, IREB2-Cor, RPLP2P4-Cor, SPTY2D1-Cor, DCUN1D5-Ups, ATXN7L1-Ups, IZUMO2-Ups, LRRC3C-Ups, VTI1BP3-Cor, WBSCR17-Cor, LOC100131510-Cor, MEN1-Cor, ZNF187-Ups, KIAA1737-Ups, LOC100507206-Ups, SLC25A11-Cor, PCBP3-Ups, FAM19A4-Cor, SLC22A13-Ups, PUS1-Cor, TRNAP19-Ups, SRP14P1-Ups, ABCA7-Ups, PKNOX2-Ups, TM4SF20-Cor, LOC100421364-Cor, MIR4518-Cor, TMEM92-Cor, MIR657-Cor, TRIM62-Cor, ODC1-Ups, RPL30P4-Ups, LOC729313-Ups, ZNF229-Cor, HS3ST2-Cor, GNAI2-Ups, SIGLEC7-Ups, NXPH4-Cor, CRBN-Cor, LOC730974-Cor, LARP1P1-Cor, RBM27-Cor, RPS12P26-Cor, LOC100130241-Ups, ACY3-Ups, UBE2CP5-Cor, TNFSF14-Ups, LOC654780-Cor, NEFM-Cor, MIR662-Cor, MAN1C1-Cor, SMARCD2-Cor, TMEM59L-Cor, C9orf62-Ups, MIR4324-Cor, PNPLA2-Cor, PCGF6-Cor, TPBGL-Cor, RPEP3-Cor, LOC100419895-Cor, ITIH2-Cor, NKX2-6-Ups, DNM1P42-Ups, MYT1L-Ups, KCNK1-Cor, LOC643308-Ups.

### Liste 31 (Kohorte 3 Prostatektomie-Tübingen):

GUCY1A3-Cor, C1R-Cor, NAGLU-Ups, UBE2CP5-Ups, HMOX1-Cor, GRIFIN-Ups, ATG2A-Cor, MEN1-Ups, EHD1-Ups, LOC100419845-Ups, ARPC4-Cor, CASKIN1-Cor, LOC390956-Ups, ARSH-Cor, SNORD88B-Cor, LOC100421504-Cor, TAF6L-Cor, ALOX15P1-Cor, TCEB3-Cor, MUC16-Cor, KIR2DL3-Cor, RNY5P6-Ups, MIR4518-Cor, KCNG4-Cor, LOC100506195-Cor, CD27-AS1-Cor, PRPSAP2-Cor, SNORD5-Cor, LOC147670-Cor, ZNF187-Ups, MIR662-Ups, EPHA10-Cor, WBSCR17-Cor, ZHX3-Ups, LOC100996289-Ups, VSTM1-Cor, STOML3-Cor, WBP11-Cor, USP35-Cor, AICDA-Cor, ADAD2-Ups, VTI1BP3-Cor, LOC100996916-Ups, ORAI3-Ups, EIF1AD-Ups, MIR548AK-Cor, NDUFB7-Cor, CEP350-Ups, ZNF607-Ups, RPL36-Ups, INSR-Ups, LOC147670-Ups, RANGRF-Cor, GRN-Ups, DENND5A-Cor, TRNAR3-Ups, YBX2-Cor, MIR4489-Ups, CTXN2-Cor, MIR3972-Ups, C1orf198-Cor, LZTS1-AS1-Cor, SYCN-Cor, KCNG4-Ups, MAPK8IP3-Cor, ZNF600-Ups, GPX1-Cor, ACRBP-Cor, LMTK3-Cor, RPS5P6-Ups, LOC115110-Cor, PITPNM1-Ups, LOC100419839-Ups, TRAPPC2P1-Ups, LOC100996630-Cor, ZNF26-Cor, KBTBD11-Ups, ASCL2-Ups, UBXN6-Ups, ZNF614-Ups, B4GALNT4-Ups, LSMD1-Ups, RPS15AP16-Ups, SYF2-Cor, MYEF2-Cor, OR52P2P-Cor, LOC100505685-Cor, LDHB-Cor, LOC100419895-Cor, CDK2AP1-Cor, RPS17P7-Ups, ATXN7L1-Ups, LY6G5C-Ups, ID3-Cor, ZNF415-Cor, OR10H4-Cor, TRNAA2-Ups, EIF4G3-Ups, AURKC-Ups, KCNK1-Cor, ACTN3-Cor, TMEM184B-Cor, ZNF614-Cor, PUS10-Cor, MIR3972-Cor, FBLN2-Ups, SNAPC2-Ups, REP15-Ups, ENO1P2-Cor, RHBDD2-Ups, EIF4E3-Cor, ZNF528-Ups, ALDH3A1-Ups, GNB2L1-Cor, CCNJP1-Cor, MIR455-Cor, OR2W2P-Ups, SMARCC2-Cor, LOC100288866-Ups, BLCAP-Cor, ZNF8-Ups, PELI3-Cor, LOC387791-Cor, TNK1-Ups, CEACAMP1-Cor, OR7E1P-Ups, MCCD1P2-Ups, AADACL2-Cor, PKD2L1-Ups, SEC61A2-Cor, BRD4-Cor, C3-Cor, LOC100506753-Cor, EIF3D-Ups, DAK-Ups, LOC654780-Cor, LOC100506710-Ups, MIR526A2-Cor, TIMM8BP2-Ups, SRP14P1-Ups, ZNF534-Cor, RPL12P42-Ups, TRNAC11-Cor, LOC100996366-Cor, CRNKL1-Ups, HIATL2-Ups, ALDH7A1P3-Cor, PIM1-Cor LOC100505754-Ups, MIR30E-Ups, PROCR-Ups, KCNC3-Cor, MT1P1-Ups, ZNF304-Ups, RPS29P24-Ups, ODC1-Ups, FNTA-Cor, MIR518B-Cor, ZNF550-Ups, ASCL2-Cor, MIR4649-Cor, PUS1-Cor, LRRC23-Ups, CYP2F1P-Ups, IZUMO2-Ups, CTXN2-Ups, CGB5-Cor, SHISA7-Cor, TNNT3-Cor, FAR1-Cor, TPI1-Ups, LRGUK-Ups, VSTM2L-Ups, LOC100419033-Ups, TNK1-Cor, LOC100422610-Cor, CALM3-Cor, PPP1R2-Ups, IMPDH1P5-Ups, TMEM254-Cor, LOC100505585-Ups, GUSBP5-Ups, PMS2-Cor, TAF6L-Ups, ANKRD12-Cor, LOC100507206-Ups, POLR2F-Ups, LOC100509484-Cor, IQCF5-Cor, LOC100130527-Ups, RPS2P48-Cor, PPFIA3-Cor, C16orf46-Ups, RBMX2-Cor, TMSB4XP2-Ups, ZFP36-Cor, CAMK1-Cor, VWC2L-Cor, LOC730144-Cor, FAM71E2-Ups, EHD1-Cor, NXPH4-Cor, SYT8-Cor, RNU5B-4P-Cor, SIGLEC7-Ups, RYBP-Cor, PFKFB3-Ups, SPRYD7-Cor, RPS26P16-Ups, CHST9-Cor, ATP1B2-Ups, MKNK1-AS1-Cor, ZBTB44-Ups, CARM1-Ups, PRAMEF12-Cor, MIR516B2-Cor, RPL21P4-Ups, NADSYN1-Ups, RRAS-Ups, RPS15AP32-Ups, IFNA17-Cor, TAF12-Ups, KCNQ4-Ups, RPL23AP18-Cor, TRNAI7-Cor, ARF1P2-Cor, TRNAS7-Ups, ZNF512-Cor, NKX3-1-Ups, ZNF132-Cor, RPL3P10-Ups, ANKRD16-Cor, HYDIN-Cor, RPL14-Cor, DHX8-Cor, LOC100131510-Cor, RPL6P24-Cor, FAM87B-Cor, ZNF788-Ups, RPL23AP32-Ups, MRPL4-Ups, ATP6V0E1P4-Ups, MYH4-Ups, ATP5G2P1-Ups, MIR4522-Cor, LOC100131536-Ups, NDUFV1-Ups, FETUB-Cor, PCMTD1P2-Cor, EIF2AP1-Cor, RPL39P17-Ups, STARD3-Cor, SYDE1-Cor, FAM90A11P-Cor, UBAC1-Ups, LHFPL3-AS1-Ups, HMGN2P2-Ups, MIR299-Cor, CDC42EP5-Ups, PPP1R9B-Ups, ZSCAN23-Ups, RBFOX3-Ups, DCAF10-Cor, MIR769-Cor, LOC100130632-Cor, SFT2D3-Cor, LOC285074-Cor, SPTY2D1-Cor, LRRC3C-Ups, C7orf65-Cor, KCTD11-Ups, C19orf38-Cor, VN1R107P-Cor, RPL30P4-Ups, LOC100507194-Cor, KLF2-Cor, ACPT-Ups, VSTM5-Ups, MIER2-Cor, PAGE4-Cor, TMEM80-Ups, LOC151174-Cor, TPM4-Cor, MRPL45P2-Cor, IL17C-Cor, RPL9P17-Ups, KDM3A-Cor, REP15-Cor, TMEM88-Cor, LOC284080-Cor, LOC100422581-Ups, LOC101060778-Ups, HNRNPA3P4-Ups, PSG8-Ups, LOC389120-Ups, PCBP3-Ups, LOC100130894-Cor, BTN3A3-Ups, LSM14B-Ups, ZNF653-Ups, OSBPL2-Ups, FAM178A-Ups, MIR4650-1-Ups, CDK2AP1-Ups, TMEM80-Cor, LOC644881-Cor, FUT5-Cor, HBG2-Ups, LOC100505863-Cor, PGBD1-Ups, MIR524-Ups, RIMKLA-Cor, LOC100507530-Ups, AUTS2-Cor, LOC100996788-Ups, MYEF2-Ups, CIB3-Cor, SNORD59A-Cor, ZNF135-Ups, APPL1-Ups, FOXRED1-Cor, MAFG-AS1-Ups, LOC100288596-Ups, LOC390203-Ups, TBX5-AS1-Ups, ANKRD52-Cor, MIR548A2-Ups, COX6A1P2-Ups, ZNF775-Ups, TPBGL-Cor, FAM87B-Ups, LOC100631378-Cor, MYH1-Ups, DDB1-Cor, ACTR5-Cor, SLC16A6-Cor, MIR516B2-Ups, TMEM160-Cor, ZNF200-Cor, LOC441320-Cor, LOC100130169-Cor, GXYLT2-Ups, LOC100420114-Ups, RPL9P29-Cor, SYF2-Ups, GPRC5A-Ups, RPS15AP32-Cor, MIR645-Ups, CIC-Ups, HYDIN-Cor, LOC648044-Ups, IFT74-Cor, LOC100509541-Ups, TEAD4-Ups, LOC145820-Ups, CCDC171-Cor, CEACAM8-Cor, LOC100420668-Cor, PRSS57-Ups, C12orf60-Cor, ZNF229-Cor, CXorf51A-Cor, OR10H4-Ups, GDF5OS-Cor, SIGLEC16-Ups, IGSF21-Ups, SLC25A39-Ups, PCBP3-Cor, NFKBIB-Cor, POLH-Cor.

### Liste 32 (Kohorte 4 Plasma-Tübingen):

NANP-Ups, TP53-Cor, MIRLET7F2-Cor, LENG1-Ups, ID3-Cor, STK38-Cor, SLC28A2-Cor, FAM19A4-Cor, ADORA2BP1-Cor, SHCBP1L-Ups, LOC401127-Ups, LOC441806-Ups, PRICKLE1-Cor, BRE-AS1-Cor, MIR194-2-Cor, RNF222-Ups, SLC52A1-Ups, OR6C2-Ups, TRNAR29-Cor, LOC100287792-Ups, FAM86GP-Ups, FLYWCH2-Cor, MICAL1-Cor, ATG2A-Cor, LOC441228-Ups, LOC100420089-Cor, USP34-Cor, NTAN1-Cor, DND1P1-Ups, MIR4493-Ups, KCNG4-Cor, MTRNR2L10-Cor, CEP76-Cor, TRAF3IP3-Ups, MAGEB1-Ups, VSTM5-Ups, PELI3-Cor, MIR548T-Cor, SNORD96A-Ups, SNORA1-Cor, TRNAY8-Ups, MTERFD3-Ups, A1BG-AS1-Cor, OR51A10P-Ups, TMF1-Cor, SNORA25-Cor, RPP25L-Ups, MAPKAPK3-Cor, QRICH1-Cor, EHD1-Ups, LOC100287290-Cor, AVIL-Cor, PCDHA8-Cor, SNORD43-Cor, TMC3-Ups, HLTF-AS1-Cor, SLC2A8-Ups, NXN-Ups, ZBED4-Cor, TMEM95-Cor, LOC729732-Cor, TRNAR29-Ups, TRNAI22-Cor, RPL23AP32-Ups, MIR548T-Ups, TCEB3-Cor, DLGAP1-AS3-Cor, FAM66E-Cor, ALDH7A1P3-Cor, RPS26-Cor, BCAR4-Ups, ZNF775-Ups, C9orf133-Cor, WIPF2-Cor, LOC100507652-Cor, MIR1289-1-Cor, RFX7-Ups, RPS26P48-Ups, NRON-Ups, LOC100421664-Cor, LOC553139-Cor, SCGB2B3P-Cor, ZNF135-Cor, PTP4A2P1-Ups, LOC391239-Ups, LOC401777-Ups, FAM222A-Ups, RNY1-Ups, CYP7B1-Cor, LOC401127-Cor, GATA1-Cor, IQCF4-Cor, SLC9A8-Cor, OR6C2-Cor, RARRES3-Ups, LOC100652885-Ups, RGL1-Cor, NUP62-Cor, ZSCAN23-Ups, MIR518D-Ups, HNRNPA1P19-Ups, MAPK8IP3-Cor, SCML1-Cor, LOC730098-Ups, LOC100130632-Ups, MIR3662-Ups, LOC151174-Cor, HIGD1AP12-Cor, ZNF132-Ups, PPIAP29-Cor, LOC100652918-Cor, OR1R1P-Cor, MIR3919-Ups, LOC100422438-Cor, OC100421580-Cor, POF1B-Ups, LOC100130632-Cor, RPS29P24-Ups, FLJ13224-Ups, GGNBP1-Ups, GPR128-Cor, OR10AE3P-Ups, GLUL-Cor, LOC100420668-Cor, CSGALNACT1-Cor, B4GALNT4-Ups, LOC729732-Ups, MIR31HG-Cor, TLX1NB-Cor, BRE-Cor, WNT8B-Cor, HIGD1AP10-Ups, GUCY1A2-Cor, TNNT3-Ups, RPS17P10-Cor, PDK4-Ups, TRNAY7-Cor, RAB3C-Cor, KRTAP3-3-Ups, TRNAS22-Cor, LINC00272-Ups, AKAP8L-Ups, ARHGEF5-Ups, MIR3692-Ups, ALDH8A1-Ups, ZNF740-Cor, SLC35A2-Cor, C5orf44-Cor, ACPT-Cor, RPS26P14-Ups, PUS1-Ups, UBE4A-Ups, MTND1P33-Cor, ZSCAN23-Cor, LINC00348-Ups, POC5-Ups, RPL21P95-Cor, ADC-Cor, ZNF304-Cor, C6orf10-Ups, ZNF12-Cor, CDKN2B-Ups, MSLN-Cor, PFDN4-Ups, KIAA1737-Ups, LDHB-Ups, OR51G1-Ups, PITPNM1-Ups, LOC402422-Ups, CD4-Cor, CCDC59-Cor, NACC2-Cor, WBP11-Cor, MIR3162-Cor, MMP26-Cor, FLRT1-Cor, LOC100127954-Cor, RPS3AP8-Cor, TRIM62-Ups, ACRBP-Cor, ATP2B2-Cor, DDX12P-Ups, LOC100422500-Cor, UBXN6-Ups, LOC729609-Cor, NT5DC3-Cor, OR52N1-Ups, DPF1-Cor, MATK-Ups, RPL21P4-Ups, SNORD5-Cor, TRGVA-Ups, NAGLU-Ups, FOCAD-Ups, ZSCAN1-Cor, SLC17A2-Cor, MIR526A2-Cor, VSTM5-Cor, OR10V2P-Cor, KIF17-Ups, TTTY11-Ups, LOC645969-Cor, TAS2R43-Ups, GIMAP8-Cor, SUSD1-Cor, KISS1R-Cor, RPS12P14-Cor, RPL5P31-Ups, LGALS1-Cor, TRNAR12-Cor, ARHGAP35-Cor, ZNF884P-Cor, ZNF8-Cor, LOC100420074-Cor, CDC42EP5-Ups, LOC100421023-Cor, SNRNP35-Ups, RASD2-Ups, CLEC7A-Ups, IQCF4-Ups, GPR137-Cor, SEC63-Cor, OR51T1-Cor, SEC63-Ups, TRNAA44P-Ups, LINC00675-Ups, LOC401317-Cor, C1R-Cor, LOC644936-Cor, LOC100996577-Cor, MIR4780-Ups, LZTS1-AS1-Ups, ZNF616-Ups, MIR891B-Cor, GUCA1B-Cor, C7orf50-Cor, ADIPOQ-Cor, LOC100128105-Cor, ACTR3BP3-Cor, MIRLET7F2-Ups, FAM131C-Cor, RPL22P14-Ups, LOC100505995-Ups, NAIP-Cor, CCL11-Ups, YBX2-Ups, FTH1P26-Ups, KLF2-Ups, VEGFA-Ups, RPLP2P4-Cor, MIR4265-Ups, LOC100507494-Ups, MIR658-Cor, ABHD6-Cor, ABTB1-Ups, KLKP1-Ups, LOC100653282-Cor, LEUTX-Cor, MARK2P1-Cor, LOC100130394-Ups, GGA2-Cor, KLK9-Cor, SPACA3-Ups, TRBV7-8-Ups, LOC100420944-Ups, SLC17A2-Ups, SULT2A1-Ups, IL17RB-Cor, RPS3AP9-Ups, MYH3-Ups, LOC100129617-Ups, LOC100421580-Ups, PKP1-Cor, OR2AK2-Ups, APEH-Ups, GAR1-Ups, DKFZp686K1684-Ups, LOC100506639-Cor, ZNF510-Cor, MARK2P1-Ups, LOC100505822-Ups, HIST1H3I-Cor, LOC100419014-Cor, DPF1-Ups, ARL14EP-Ups, LRRC3C-Cor, MIR548AI-Cor, LOC100887067-Cor, DKK4-Ups, TMPRSS9-Cor, LOC100129434-Ups, ZAK-Cor, KIR2DL3-Cor, CCDC144B-Ups, RAB3B-Cor, OR52J2P-Cor, MRPS10P5-Cor, PRRC2A-Ups, TMEM106A-Ups, LILRA5-Ups, LRRC6-Cor, RPL3P10-Cor, RNU7-82P-Cor, FCGRT-Ups, FBXO18-Ups, BCL2L15-Cor, STARD3-Cor, RPL35P8-Cor, TRNAQ44P-Cor, PCBP3-Cor, MIR4302-Ups, TAS2R64P-Cor, IMPDH1P5-Ups, LDHB-Cor, KCTD11-Cor, LOC100129566-Ups, IFFO2-Cor, LOC442389-Cor, LOC100288728-Cor, OR6U2P-Cor, ADAM5-Ups, C16orf58-Ups, IGSF21-Ups, SUN1-Cor, MTRNR2L6-Ups, SIT1-Ups, OR51B8P-Cor, SYCP1-Cor, TEX101-Ups, CHD1-Ups, LOC100420772-Ups, LOC389120-Cor, FLJ45340-Cor, LOC100507223-Cor, KCNQ4-Ups, RPL15P7-Cor, OR52J2P-Ups, A1CF-Ups, FYTTD1P1-Ups, KCNG4-Ups, NT5DC3-Ups, SH2D3A-Cor, ANKRD16-Ups, TIGIT-Cor, TRNAW4-Cor, MIR192-Cor, LOC441806-Cor, KDM3A-Cor, RNU5B-4P-Cor, GUCY1A2-Ups, RNU7-18P-Ups, ARL6IP5-Cor, DDX42-Cor, GNA11-Ups, SHC1P2-Cor, FAM18A-Ups, LOC390614-Ups, ALDH8A1-Cor, TRNASUP1-Cor, CCDC141-Ups, LOC729327-Ups, ZNF512-Cor, PIAS3-Ups, LOC100996505-Cor, CYP4Z2P-Cor, DMD-Cor, MIR30C1-Cor, KLF2-Cor, NAV2-AS4-Ups, IER5-Cor, HIST1H1T-Ups, RPL26P19-Cor, GPR125-Cor, LOC100506620-Cor, LEUTX-Ups, SLC36A3-Cor, USP6NL-Cor, MIR520B-Cor, SNN-Ups, NDUFB8P2-Cor, PTGES3L-AARSD1-Cor, NKTR-Cor, RPL31P22-Cor, DNM1P37-Cor, OR6C73P-Cor, SLC20A1-Cor, MIR22-Cor, TAF4-Ups, LOC642614-Ups, TIGD1-Cor, GJA4-Ups, LOC100507600-Cor, RPL9P17-Ups, EEF1D-Cor, MIR507-Cor, MIR194-2-Ups, FETUB-Ups, METTL25-Cor, LOC442112-Cor, OR4D7P-Ups, RPS19BP1-Ups, RPS12P10-Ups, LOC126860-Ups, FRG2B-Cor, RPS2P25-Ups, GOLGA6L4-Cor, MAGI2-AS2-Cor, CYTH3-Cor, RAD54L-Ups, C18orf56-Cor, RNU7-13P-Cor, DHX8-Cor, GLIS1-Ups, MIR1284-Ups, ZNF419-Cor, HEATR1-Cor, RNPEPL1-Ups, LOC100420772-Cor, FAM153C-Ups, TRNAR7-Cor, EEF2K-Ups, OR52P2P-Cor, RPL23AP18-Cor, ALAD-Ups, SNRPGP3-Ups, BRE-AS1-Ups, AUNIP-Cor, SNORA8-Cor, HIST1H2APS3-Cor, LOC100287632-Cor, OR52H1-Cor, RN7SKP6-Cor, PCDHB16-Cor, MIR4428-Cor, AP2A1-Ups, ARID3B-Ups, TTLL3-Cor, MUTYH-Cor, C2orf65-Cor, ACP1-Cor, CNOT8-Ups, LOC553139-Ups, RRP8-Cor, FSTL3-Ups, HS3ST2-Ups, KIDINS220-Cor, MIR302E-Ups, PCDHB7-Ups, SLC16A6-Cor, OR51A2-Cor, SNX11-Ups, EIF1AD-Ups, RIMS4-Cor, CACNA1E-Cor, GPR146-Cor, LOC100128398-Cor, C6orf70-Cor, FAM54A-Cor, H1FX-Cor, COX6A1P2-Ups, KIR2DL3-Ups, SNRPGP8-Ups, UQCRC2P1-Ups, OR52A1-Ups, ARL4A-Cor, ASIC2-Cor, ZNF599-Ups, ALB-Cor, VTI1BP3-Cor, TCTE3-Cor, RPL35P8-Ups, OR5AN2P-Ups, HYALP1-Ups, IFNA12P-Ups, ASS1P13-Cor, PIM1-Cor, FADD-Ups, PCDHAC2-Cor, LOC100288974-Ups, LOC100653000-Cor, RPL9P29-Cor, GPX1-Cor, RARA-Cor, CRBN-Cor, ATIC-Cor, RNU7-18P-Cor, FOXF2-Cor, ARHGAP35-Ups, LOC391448-Cor, FAM19A1-Cor, MED20-Cor, PCBP3-Ups, TRBV10-3-Cor, TMUB2-Cor, FAM129A-Cor, PCGF6-Ups, PLCD3-Cor, TMEM229B-Cor, PRB3-Cor, OTP-Cor, PRSS36-Ups, AK1-Ups, RPS15AP36-Cor, THEM4P1-Ups, ZFY-Cor, HAUS6-Ups, OR1L3-Cor, NDUFA3-Ups, PGAM1P5-Cor, MIR513C-Cor, LRRC19-Cor, LRRC14B-Cor, ABCA8-Cor, ANXA13-Cor, MIR3162-Ups, CRYGEP-Cor, PTTG1IP-Ups, LOC100287792-Cor, LY6G5C-Ups, TRNAT22-Ups, KRBOX1-Ups, KIN-Cor, PLCXD3-Ups SRPR-Cor, LRRC3C-Ups, LOC100420668-Ups, LOC100190922-Cor, MIA-RAB4B-Cor, RPS13P6-Cor, SPSB2-Cor, CHST9-Ups, MAPT-Ups, IFNA1-Cor, HMGB3P5-Cor, VAC14-Ups, HCN2-Cor, STIP1-Cor, IFT74-Cor, AIFM1-Ups, DENND5A-Ups, APPL1-Ups, SNORD35A-Cor, NPTXR-Cor, DDA1-Cor, DDX12P-Cor, ANKRD16-Cor, TRNAA2-Ups, CYP4F11-Ups, RPL21P54-Cor, KCNK1-Cor, LOC285453-Ups, BTN1A1-Ups, KIF2C-Ups, LUC7L-Cor, RPL12P42-Ups, TRNAR3-Cor, ABT1-Cor, ZNF586-Ups, MPC1L-Cor, RPS15AP8-Cor, LRRC43-Cor, CELSR3-Ups, ITGB8-Ups, VWC2L-Cor, MYLK4-Ups, LOC100420944-Cor, LOC285453-Cor, RPL10AP5-Cor, EHD2-Cor, DSTN-Cor, ZNF233-Ups, EPHX3-Cor, FAM90A11P-Ups, OR4D6-Cor, FP588-Cor, CEACAM18-Cor, HADH-Cor, ZNF90P1-Ups, LOC727964-Ups, FAM71E2-Ups, SIGMAR1-Cor, GSDMB-Ups, BPI-Ups, OR51A8P-Cor, HMGB3P2-Ups, ATF5-Cor, LPIN1-Ups, OR4A11P-Cor, RPL7P46-Ups, GMFBP1-Ups, LOC642652-Ups, TAS2R43-Cor, TUB-Cor, C10orf131-Ups, NACC1-Ups, OR51A3P-Cor, PPDPF-Cor, LOC100421525-Ups, LOC100128136-Ups, SPTAN1-Cor, OR8B3-Cor, CLEC7A-Cor, SNAPC5-Ups, C17orf59-Ups, ATP5J2P5-Cor, HBBP1-Cor OR5A2-Cor, ACSM2B-Cor, LOC100507521-Ups, SPPL2C-Cor, RPL34P11-Cor, LOC100507073-Ups, LOC100507194-Ups, FABP3P2-Cor, MIR892B-Ups, GSE1-Cor, CHD7-Cor, LGALS13-Ups, ERCC1-Ups, LYRM7-Cor, CYP4F36P-Ups, MMP24-Cor, CTXN2-Cor, LOC100421695-Cor, OGFOD2-Cor, LOC390099-Cor, SEMA5B-Cor, RPL15P6-Cor, ABCA13-Cor, LOC100132356-Cor, PKD2L1-Ups, LOC100505702-Ups, HSBP1-Cor, ADAM11-Cor, PGBD1-Ups, FLRT1-Ups, LOC728769-Ups, RPL39P7-Cor, ABCA8-Ups, MTHFD2L-Ups, MYH3-Cor, BCL6-Cor, AP3S2-Cor, STARD3-Ups, MT1P2-Cor.

### Liste 33 (Schnittmenge der Kohorten 1.2):

WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, LOC401317-Cor, CRNKL1-Ups, MCCD1P2-Ups, DSG1-Cor, SLC22A24-Ups, ZNF26-Cor, NXPH4-Cor, MEN1-Ups, KDM3A-Cor, ARHGAP35-Cor, TRNAA2-Ups, EIF1AD-Ups, ACTN3-Cor, SPTY2D1-Cor, ASCL2-Ups, ZNF132-Cor, HMOX1-Cor, PPP1R2-Ups, WBSCR17-Cor, ARSH-Cor, ALDH7A1P3-Cor, NAGLU-Ups, ACRBP-Cor, ZNF304-Ups, MIR1972-2-Ups, TMEM33-Ups, LOC100419839-Ups, C1R-Cor, SNORD88B-Cor, NDUFB7-Cor, NTMT1-Ups, BRD4-Cor, LOC100131510-Cor, TIMM8BP2-Ups, KCNK1-Cor, RIMKLA-Cor, RPL12P42-Ups, PCGF6-Cor, LOC100419895-Cor, IZUMO2-Ups, PCBP3-Ups, ASS1P13-Cor, LOC100419848-Ups, LZTS1-AS1-Cor, LOC145757-Cor, PPFIA3-Cor, TRNAV20-Ups, LARP1P1-Cor, ZNF187-Ups, MIR4518-Cor, LOC100130394-Ups, ACPT-Cor, RPL23AP18-Cor, ODF2L-Cor, C16orf46-Ups, LOC100422241-Ups.

Die Gen-Abschnitte der Schnittmenge der Kohorten 1, und 2 eignen sich bevorzugt für eine frühe Diagnose eines Tumors, insbesondere eines Prostatatumors.

### Liste 34 (Schnittmenge der Kohorten 1.3):

WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, CRNKL1-Ups, ZHX3-Ups, RPL23AP32-Ups, MCCD1P2-Ups, ANKRD12-Cor, CD27-AS1-Cor, ZNF26-Cor, NXPH4-Cor, MEN1-Ups, KDM3A-Cor, TRNAA2-Ups, EIF1AD-Ups, TPI1-Ups, ACTN3-Cor, KCNG4-Ups, GUSBP5-Ups, SPTY2D1-Cor, LOC147670-Cor, HYDIN-Cor, PKD2L1-Ups, ASCL2-Ups, ZNF132-Cor, GUCY1A3-Cor, HMOX1-Cor, PPP1R2-Ups, WBSCR17-Cor, ARSH-Cor, ALDH7A1P3-Cor, NAGLU-Ups, ACRBP-Cor, ZNF304-Ups, GRIFIN-Ups, MIR548AK-Cor, YBX2-Cor, LOC100419839-Ups, C1R-Cor, SNORD88B-Cor, NDUFB7-Cor, HNRNPA3P4-Ups, BRD4-Cor, ATG2A-Cor, LOC100131510-Cor, CDC42EP5-Ups, TIMM8BP2-Ups, KCNK1-Cor, RIMKLA-Cor, RPL12P42-Ups, HBG2-Ups, DDB1-Cor, FAM90A11P-Cor, LOC100419895-Cor, RPL9P17-Ups, IZUMO2-Ups, PCBP3-Ups, TBX5-AS1-Ups, ZNF415-Cor, LZTS1-AS1-Cor, AURKC-Ups, EPHA10-Cor, PPFIA3-Cor, KBTBD11-Ups, ZNF187-Ups, LOC100996788-Ups, ORAI3-Ups, LOC100507530-Ups, VSTM2L-Ups, ACTR5-Cor, MIR4518-Cor, LOC284080-Cor, RPL23AP18-Cor, LOC100996366-Cor, MIR526A2-Cor, MIR516B2-Cor, LOC100130632-Cor, CIB3-Cor, CHST9-Cor, ZNF788-Ups, ANKRD16-Cor, EIF4E3-Cor, CTXN2-Cor, C16orf46-Ups, LOC100631378-Cor, RPS2P48-Cor, FAM87B-Ups, LOC644881-Cor, TRNAS7-Ups, LSMD1-Ups.

Die Gen-Abschnitte der Schnittmenge der Kohorten 1, und 3 eignen sich bevorzugt für eine frühe Diagnose eines Tumors, insbesondere eines Prostatatumors.

### Liste 35 (Schnittmenge der Kohorten 2.31:

LOC390203-Ups, ZNF26-Cor, WBP11-Cor, ARPC4-Cor, TAF6L-Cor, NDUFB7-Cor, KIR2DL3-Cor, UBXN6-Ups, HMOX1-Cor, MEN1-Ups, TAF6L-Ups, PROCR-Ups, LOC100996630-Cor, OR52P2P-Cor, ACRBP-Cor, RNU5B-4P-Cor, MIR548A2-Ups, BRD4-Cor, ASCL2-Cor, ASCL2-Ups, FOXRED1-Cor, MAPK8IP3-Cor, LOC100419839-Ups, TRNAA2-Ups, PIM1-Cor, TRNAR3-Ups, C1R-Cor, C1R-Cor, RPL23AP18-Cor, HMGN2P2-Ups, EIF1AD-Ups, MIR3972-Ups, B4GALNT4-Ups, ALDH7A1P3-Cor, LHFPL3-AS1-Ups, PPP1R2-Ups, ALOX15P1-Cor, KDM3A-Cor, SNORD88B-Cor, ZNF528-Ups, RPS15AP32-Ups, ACTN3-Cor, MIR516B2-Ups, RPL12P42-Ups, FUT5-Cor, TIMM8BP2-Ups, GPX1-Cor, C7orf65-Cor, RNY5P6-Ups, C16orf46-Ups, MCCD1P2-Ups, SYDE1-Cor, ZNF132-Cor, CRNKL1-Ups, LOC389120-Ups, LZTS1-AS1-Cor, CDK2AP1-Cor, ARSH-Cor, MIR518B-Cor, LOC100506753-Cor, RIMKLA-Cor, PPFIA3-Cor, NAGLU-Ups, ZNF304-Ups, REP15-Ups, EIF4G3-Ups, HIATL2-Ups, ZNF534-Cor, LOC115110-Cor, SPTY2D1-Cor, ATXN7L1-Ups, IZUMO2-Ups, LRRC3C-Ups, VTI1BP3-Cor, WBSCR17-Cor, LOC100131510-Cor, ZNF187-Ups, LOC100507206-Ups, PCBP3-Ups, PUS1-Cor, SRP14P1-Ups, MIR4518-Cor, ODC1-Ups, RPL30P4-Ups, ZNF229-Cor, SIGLEC7-Ups, NXPH4-Cor, LOC654780-Cor, TPBGL-Cor, LOC100419895-Cor, KCNK1-Cor.

Die Gen-Abschnitte der Schnittmenge der Kohorten 2 und 3 eignen sich bevorzugt für eine frühe Diagnose eines Tumors, insbesondere eines Prostatatumors.

### Liste 36 (Schnittmenge der Kohorten 3.4):

C1R-Cor, NAGLU-Ups, ATG2A-Cor, EHD1-Ups, TCEB3-Cor, KIR2DL3-Cor, KCNG4-Cor, SNORD5-Cor, WBP11-Cor, VTI1BP3-Cor, EIF1AD-Ups, CTXN2-Cor, KCNG4-Ups, MAPK8IP3-Cor, GPX1-Cor, ACRBP-Cor, PITPNM1-Ups, UBXN6-Ups, B4GALNT4-Ups, OR52P2P-Cor, LDHB-Cor, LY6G5C-Ups, ID3-Cor, TRNAA2-Ups, KCNK1-Cor, PELI3-Cor, PKD2L1-Ups, MIR526A2-Cor, RPL12P42-Ups, ALDH7A1P3-Cor, PIM1-Cor, RPS29P24-Ups, IMPDH1P5-Ups, VWC2L-Cor, FAM71E2-Ups, RNU5B-4P-Cor, RPL21P4-Ups, KCNQ4-Ups, RPL23AP18-Cor, ZNF512-Cor, ANKRD16-Cor, DHX8-Cor, RPL23AP32-Ups, STARD3-Cor, CDC42EP5-Ups, ZSCAN23-Ups, LOC100130632-Cor, LRRC3C-Ups, KLF2-Cor, VSTM5-Ups, LOC151174-Cor, RPL9P17-Ups, KDM3A-Cor, PCBP3-Ups, PGBD1-Ups, APPL1-Ups, COX6A1P2-Ups, ZNF775-Ups, SLC16A6-Cor, RPL9P29-Cor, IFT74-Cor, LOC100420668-Cor, IGSF21-Ups, PCBP3-Cor.

Die Gen-Abschnitte der Schnittmenge der Kohorten 3 und 4 eignen sich bevorzugt für eine frühe Diagnose eines Tumors, insbesondere eines Prostatatumors.

### Liste 37 (Schnittmenge der Kohorten 1. 2. 3):

WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, CRNKL1-Ups, MCCD1P2-Ups, ZNF26-Cor, NXPH4-Cor, MEN1-Ups, KDM3A-Cor, TRNAA2-Ups, EIF1AD-Ups, ACTN3-Cor, SPTY2D1-Cor, ASCL2-Ups, ZNF132-Cor, HMOX1-Cor, PPP1R2-Ups, WBSCR17-Cor, ARSH-Cor, ALDH7A1P3-Cor, NAGLU-Ups, ACRBP-Cor, ZNF304-Ups, LOC100419839-Ups, C1R-Cor, SNORD88B-Cor, NDUFB7-Cor, BRD4-Cor, LOC100131510-Cor, TIMM8BP2-Ups, KCNK1-Cor, RIMKLA-Cor, RPL12P42-Ups, LOC100419895-Cor, IZUMO2-Ups, PCBP3-Ups, LZTS1-AS1-Cor, PPFIA3-Cor, ZNF187-Ups, MIR4518-Cor, RPL23AP18-Cor, C16orf46-Ups.

Die Gen-Abschnitte der Schnittmenge der Kohorten 1, 2 und 3 eignen sich bevorzugt für eine frühe Diagnose eines Tumors, insbesondere eines Prostatatumors.

### Liste 38 (Schnittmenge der Kohorten 1. 2. 3. 4):

WBP11-Cor, B4GALNT4-Ups, UBXN6-Ups, KIR2DL3-Cor, OR52P2P-Cor, KDM3A-Cor, TRNAA2-Ups, EIF1AD-Ups, ALDH7A1P3-Cor, NAGLU-Ups, ACRBP-Cor, C1R-Cor, KCNK1-Cor, RPL12P42-Ups, PCBP3-Ups, RPL23AP18-Cor.

Die Gen-Abschnitte der Schnittmenge der Kohorten 1, 2, 4 und 4 eignen sich bevorzugt für eine frühe Diagnose eines Tumors, insbesondere eines Prostatatumors.

Bei den zuvor in den Listen genannten Gen-Abschnitten handelt es sich um regulatorische 5' Sequenzabschnitte der genannten Gene. Cor ist definiert als der 5'-Bereich des jeweiligen Gens, der den Sequenzabschnitt zwischen der Nukleotidposition -500 stromaufwärts und der Nukleotidposition 500 stromabwärts des Transkriptionsstarts beinhaltet. Ups ist definiert als der 5'-Bereich des jeweiligen Gens, der den Sequenzabschnitt zwischen der Nukleotidposition -1500 stromaufwärts und der Nukleotidposition -500 stromaufwärts des Transkriptionsstarts beinhaltet.

Der zuvor verwendete Begriff "Biomarker" hat auch die Bedeutung von Tumormarker. Die Bezeichnungen "hypermethyliert" und "hypomethyliert" bezeichnen den Methylierungszustand im Vergleich zu einer Kontrollgruppe mit nichterkrankten Probanden.

Die oben in den Listen genannten Gen-Abschnitte und deren Nukleotidsequenzen sind in der Datenbank Gene (Brown G, Wallin C, Tatusova T, et al., Gene Help: Integrated Access to Genes of Genomes in the Reference Sequence Collection, 2006 Sep 13, updated 2013 Dec 2,. in Gene Help [Internet], Bethesda (MD): National Center for Biotechnology Information (US); 2005, http://www.ncbi.nlm.nih.gov/books/NBK3841/ beschrieben.

Erfindungsgemäß wird innerhalb eines DNA-Abschnitts gemessen, d.h. dass mindestens ein differentiell methyliertes Cytosin relativ zum Vorkommen des DNA-Abschnitts in der Probe gemessen wir. Die Messung bezieht sich damit auf den DNA-Abschnitt selbst in der Normierung. Damit unterscheidet sich das Verfahren wesentlich von Verfahren des Stands der Technik, bei denen eine Normierung auf einen DNA-Abschnitt bezogen wird, der sich beispielsweise auf einem Plasmid befindet oder im selben Genom, aber auf einem anderen Chromosom oder auf dem gleichen Chromosom, aber an einem anderen Genlocus.

Ein bevorzugtes Verfahren zur Bestimmung des Vorliegens einer differentiellen Methylierung eines Cytosins basiert auf der Bisulfitierung von DNA mit anschließender Analyse der erzeugten bisulfitierten DNA.

Um von isolierter DNA zu Bisulfit-konvertierter DNA zu gelangen, wird diese durch eine dem Fachmann hinlänglich bekannte Bisulfitierungsreaktion umgewandelt. Hierbei werden die unmethylierten Cytosine der DNA durch das Bisulfit in Uracil umgewandelt. Als Folge der Umwandlung können verschiedene Varianten einer umgewandelten Nukleinsäure vorliegen, die von der Anzahl der unmethylierten Cytosine dieser Nukleinsäure abhängen. Eine Nukleinsäure, die beispielsweise 2 Cytosine enthält kann demnach, je nach Methylierungszustand dieser Cytosine, zu 4 unterschiedlichen Varianten nach der Bisulfitierung führen, da entweder keines, das erste, das zweite, oder beide der Cytosine unmethyliert vorliegen kann/können und in Uracil umgewandelt wird/werden. Diese unterschiedlichen Varianten können in einer bevorzugten Ausführungsform mittels spezifischer Primer oder Primerpaare detektiert werden.

An die Umwandlung kann zunächst sich in einer Ausführungsform ein weiterer Schritt der Aufreinigung der bisulfitierten DNA anschließen. In einer weiteren Ausführungsform wird die Bisulfitierung der DNA als weiterer Schritt von dem Verfahren der Erfindung umfasst, als Bestandteil der quantitativen Bestimmung des Vorliegens eines differentiell methylierten Cytosins eines CpG Dinukleotids.

In einer weiteren, besonders bevorzugten Ausführungsform umfasst Schritt a) die Amplifikation des DNA-Abschnitts mit zumindest einem Primerpaar, das spezifisch für den DNA-Abschnitt ist, wobei die Primer bevorzugt keine möglicherweise differentiell methylierte Position des DNA-Abschnitts umfassen, d.h. kein C in einem CpG-Dinukleotid. Weiterhin umfasst Schritt b) die Amplifikation der bisulfitierten DNA mit zumindest einem Primerpaar, das spezifisch für den DNA-Abschnitt ist (welcher in Schritt a) bestimmt wurde) und das zumindest einen Primer umfasst, der zumindest eine differentiell methylierte Position des DNA-Abschnitts umfasst, d.h. zwischen zumindest einem C eines methylierten CpG und zumindest einem U/T eines bisulfitierten unmethylierten CpG diskriminieren kann.

Grundsätzlich kann auch Schritt a) mit bisulfitierter DNA durchgeführt werden, da dies nicht zu einer Veränderung der DNA-Sequenz im amplifizierten Bereich führen kann, wenn keine möglicherweise differentiell methylierte Position des DNA-Abschnitts umfasst wird.

In anderen Worten kann für die Bestimmung in Schritt a) die DNA entweder nichtbisulfitiert (also z.B. direkt nach der Isolation aus einer Probe) oder bereits bisulfitiert vorliegen. Im ersten Fall können beliebige für das Transposon spezifische Primer zu dessen Amplifizierung verwendet werden; in letzterem Fall sollte dafür Sorge getragen werden, dass die verwendeten Primer keine differentiell methylierte Stelle umfassen (also kein Cytosin eines CpG Dinukleotids). In einer bevorzugten Ausführungsform wird in den Schritten a) und b) das gleiche Volumen von DNA eingesetzt, dabei ist die DNA bevorzugt in einem Ansatz aus einer Probe isoliert worden. Noch bevorzugter werden in Schritt a) und b) gleiche Mengen an DNA eingesetzt. Auch hierbei ist die DNA bevorzugt in einem Ansatz aus einer Probe isoliert worden.

In Schritt b), der immer mit bisulfitierter DNA ausgeführt wird, ist dies genau umgekehrt; hier muss zumindest ein Primer zumindest eine differentiell methylierte Stelle des in Schritt a) amplifizierten DNA-Abschnitts umfassen (also zumindest ein Cytosin eines CpG Dinukleotids). Es spielt hierbei keine Rolle, ob der zumindest eine Primer für die zumindest eine differentiell methylierte Stelle auf dem sense oder dem antisense Strang der DNA spezifisch ist. Über diese oder diesen Primer kann somit eine an der untersuchten gegebenen Position bestehende oder nicht bestehende Methylierung der DNA nachgewiesen werden. Wird ein Amplifikat mit Primern erhalten, die spezifisch für ein CpG sind, so lag an der betreffenden Stelle eine Methylierung der ursprünglichen DNA vor, da keine Umwandlung bei der Bisulfitierungsreaktion erfolgte. Wird ein Amplifikat mit Primern erhalten, die spezifisch für ein bisulfitiertes CpG sind, so lag an der betreffenden Stelle keine Methylierung (eine Demethylierung) der ursprünglichen DNA vor. Demgemäß kann - je nach der Art der verwendeten Primer - die Methylierung oder die Demethylierung nachgewiesen werden.

Es kann für die erfindungsgemäße Bestimmung des Methylierungsgrads entweder die Methylierung oder die Demethylierung DNA-Abschnitts bestimmt werden, da diese beiden Zustände direkt miteinander korrespondieren. Wird demnach ein CpGspezifisches Primerpaar verwendet, so wird der DNA-Methylierungsgrad über die Methylierung bestimmt; wird ein für ein bisulfitiertes CpG spezifisches Primerpaar verwendet, so wird der DNA-Methylierungsgrad über die Demethylierung bestimmt.

In einer bevorzugten Ausführungsform ist zumindest ein Primer für zumindest eine differentiell methylierte Position des DNA-Abschnitts spezifisch; noch bevorzugter sind beide Primer für zumindest eine differentiell methylierte Position des DNA-Abschnitts spezifisch. Dies hat den Vorteil, dass eine bessere Spezifität und verbesserte Amplifikation erreicht wird.

In weiteren bevorzugten Ausführungsformen sind die verwendeten Primer für mehr als eine differentiell methylierte Position spezifisch, d.h. sie umfassen in ihrer Sequenz einen DNA-Bereich, der mehrere differentiell methylierte C umfasst. Solche Primer sind für mehr als ein Cytosin eines CpG bzw. bisulfitierten CpG spezifisch. In besonders bevorzugten Ausführungsformen sind die Primer für 2, 3, 4 oder mehr als 4 differentiell methylierte Position spezifisch.

Typischerweise sind die verwendeten Primer wenigstens 15 Nukleotide lang, bevorzugt 18, 19, 20, 21, 22, 23, 24, 25 oder mehr als 25 Nukleotide lang. Ein Primerpaar kann Primer mit verschiedener Länge umfassen. In einer bevorzugten Ausführungsform sind die Primer 18 bis 35 Nukleotide lang, in einer weiteren bevorzugten Ausführungsform sind die Primer 20-30 Nukleotide lang.

In einer bevorzugten Ausführungsform sind die Primer eines Primerpaars entweder ausschließlich für zumindest ein Cytosin eines CpG Dinukleotids oder ausschließlich für zumindest ein Cytosin eines bisulfitierten CpG Dinukleotids spezifisch.

Die Primer können die zumindest eine differentiell methylierte Position an jeder Stelle umfassen, d.h. am 5'-Ende des Primeroligonukleotids, am 3'-Ende oder an jeder Position dazwischen. In einer besonders bevorzugten Ausführungsform liegt das zumindest eine, für eine differentiell methylierte Position spezifische Nukleotid am 3'-Ende des Primers. Dies hat den Vorteil einer erhöhten Spezifität.

In einer weiteren bevorzugten Ausführungsform haben die Primer eines Primerpaars einen nahezu identischen Tm, bevorzugt einen Tm, der ≤ 3° C, ≤ 2° C, ≤ 1° C, ≤ 0.5° C, ≤ 0.2° C oder ≤ 0.1° C voneinander abweicht.

In einer weiteren bevorzugten Ausführungsform haben die von den Primern eingeschlossenen Sequenzbereiche eine Länge von ≥ 1 und ≤ 3000 bp, bevorzugter ≥ 10 und ≤ 2000 bp, noch bevorzugter ≥ 30 und ≤ 800 bp und am bevorzugtesten ≥ 50 und ≤ 300 bp.

Der Fachmann wird erkennen, dass nicht nur ein Primerpaar eingesetzt werden kann, sondern auch eine Vielzahl derselben. In einer weiteren Ausführungsform wird das Verfahren daher mit 2, 3, 4, 5 oder mehr als 5 Primerpaaren durchgeführt, die spezifisch für einen DNA-Abschnitt sind und die jeweils zumindest einen Primer umfassen, der für zumindest ein Cytosin eines CpG Dinukleotids oder ein bisulfitiertes Cytosin eines CpG Dinukleotid spezifisch ist. Bevorzugt haben diese mehreren Primerpaare einen nahezu identischen Tm, also bevorzugt einen Tm, der ≤ 3° C, ≤ 2° C, ≤ 1° C, ≤ 0.5° C, ≤ 0.2° C oder ≤ 0.1° C voneinander abweicht.

Die Bestimmung des normierten DNA-Methylierungsgrads wird aus den in Schritt a) und b) erhaltenen quantitativen Werten erhalten, beispielsweise durch Subtraktion oder - bevorzugt - durch Division, insbesondere des Wertes aus Schritt b) durch den Wert aus Schritt a).

Im Fall der Verwendung von Amplifikation zur quantitativen Bestimmung in den Schritten a) und b) kann der Methylierungsgrad über das Verhältnis der in den beiden Amplifikationsschritten (Schritt a) und b)) gebildeten Amplifikate bestimmt werden. In besonderen Ausführungsformen wird das Verhältnis über die gebildeten Mengen an Amplifikat bestimmt, bevorzugter über die Zunahme des pro Amplifikations-Zyklus gebildeten Amplifikats, noch bevorzugter über den cycle threshold (ct) Wert während einer real time PCR.

In einer Ausführungsform wird die Gesamtmenge der beiden gebildeten Amplifikate nach einer gleichen Anzahl von Amplifikationszyklen bestimmt und miteinander in Beziehung gesetzt. Für die Bestimmung der gebildeten Amplifikate sind dem Fachmann eine Reihe von unterschiedlichen Verfahren bekannt, einschließlich spektroskopischer Verfahren, der Anfärbung mittels Ethidiumbromid oder Silber und densitometrischer Bestimmung, oder der radioaktiven Markierung mit anschließender Bestimmung über, z.B., Szintillationsmessung.

In einer bevorzugten Ausführungsform erfolgt die Bestimmung der in den beiden Amplifikationsschritten gebildeten Amplifikate mit Hilfe der real time PCR während der Bildung der Amplifikate selbst. In einer weiteren bevorzugten Ausführungsform erfolgt die Bestimmung der in den beiden Amplifikationsschritten gebildeten Amplifikate simultan während einer Real Time PCR.

Nach der Bestimmung der in beiden Amplifikationsschritten gebildeten Amplifikate wird der Wert für das Amplifikat im zweiten Amplifikationsschritt (Schritt b)) mittels des Werts für das Amplifikat im ersten Amplifikationsschritts (Schritt a)) normiert; z.B. durch Division oder Subtraktion der ermittelten Werte. Hierdurch wird ein normierter Methylierungsgrad bestimmt (d.h. die normierte (De)-Methylierung).

In einer Ausführungsform erfolgt die Amplifikationen der Schritte a) und b) sowie die Bestimmung der gebildeten Amplifikate mittels der real time PCR. Hierbei werden cycle threshold (ct) Werte bestimmt, sowohl für das Primerpaar, welches für zumindest eine differentiell methylierte Position des DNA-Abschnitts spezifisch ist (Schritt b), ctm), als auch für das Primerpaar, welches für einen nicht differentiell methylierten Bereich des DNA-Abschnitts spezifisch ist (Schritt a), ctk). Der ct-Wert beschreibt den Zyklus der PCR, in dem das Fluoreszenzsignal erstmals signifikant über die Hintergrundfluoreszenz ansteigt und markiert damit den Anfang der exponentiellen Phase der PCR.

Danach wird der ct-Wert aus Schritt a) vom ct-Wert aus Schritt b) abgezogen, um zum normierten Methylierungsgrad (Δct) zu gelangen. Δct lässt sich also berechnen als: Δct = ctm - ctk. Ist die differentiell methylierte Position komplett methyliert und es werden methylierungsspezifische Primer verwendet, wird der Wert von ctm und ctk im Wesentlichen identisch sein. Ist ein Bereich im Wesentlichen nicht methyliert, wird er erst bei einem späteren Zyklus den ct-Wert erreichen, also einen höheren ctm-Wert aufweisen, so dass Δct positiv wird.

In Ausführungsformen der Erfindung ist es auch möglich, zunächst eine Abtrennung methylierter Bereiche vorzunehmen. Hierzu wird die Probe genomischer DNA fragmentiert. Mittels methylierungsspezifischer Antikörper werden methylierte Fragmente ausgefällt und abgetrennt. Da in diesem Fall keine methylierten Cytosine mehr vorhanden sind, kann auf die Bisulfitreaktion verzichtet werden. Es genügt, in einer Reaktion die quantitative Bestimmung der unbehandelten Probe im Schritt a) und eine quantitative Bestimmung der unbehandelten Probe im Schritt b) vorzunehmen.

Alternativ zu den genannten Bestimmungsverfahren kann auch eine methylierungsspezifische Sequenzierung erfolgen. Hierbei kann exakt ermittelt werden, in welchem Umfang eine spezifische Position methyliert oder unmethyliert ist. Das grundsätzliche Verfahren ist dem Fachmann bekannt.

Soweit die Bestimmung in den Schritten a) und b) mittels Amplifikation erfolgt, liegt bevorzugt der Abstand zwischen dem Template für die Bestimmung in Schritt a) und dem Template für die Bestimmung in Schritt b) bei nicht mehr als 250 Mio. Basenpaaren, bevorzugt bei nicht mehr als 100.000 Basenpaaren, noch bevorzugter bei nicht mehr als 10.000 oder 1.000 Basenpaaren.

In einer bevorzugten Ausführungsform wird eine nested PCR verwendet. Bei einer nested PCR werden zwei PCR-Reaktionen nacheinander durchgeführt.

In der ersten PCR wird der gesuchte PCR-Abschnitt amplifiziert und in der zweiten PCR werden die so erzeugten Abschnitte für die zweite PCR verwendet, in dem Primer verwendet werden, die einen kleineren Bereich amplifizieren, d.h. "downstream" von den ersten Primern liegen. Typisch sind eine Voramplifikation von 2-30 Zyklen mit Primern, die spezifisch sind für die methylierte Zielsequenz und eine daraus erfolgende Folgeamplifikation mit Primern, die innerhalb von 1- 500 Nukleotiden stromabwärts der Primerbindestellen der voramplifizierenden Primern liegen.

In anderen Ausführungsformen der Erfindung können beispielsweise gleichzeitig zwei oder mehr differentiell methylierte Bereiche eines DNA-Abschnitts bestimmt werden. Daraus lässt sich neben der spezifischen Aussage über die differentielle Methylierung eines speziellen Sequenzabschnitts des DNA-Abschnitts auch ein gemittelter Methylierungsgrad über den gesamten DNA-Abschnitt bestimmen.

In einer anderen Ausführungsform wird vor der Bestimmung in den Schritten a) und b) eine whole genome amplification durchgeführt. Whole genome amplifications eignen sich für Untersuchungen, bei denen nur geringe Mengen an DNA vorliegen. Wenn zwei Proben verglichen werden sollen, ist es erforderlich, dass beide eine effiziente Amplifikation erfahren; eventuelle Ungleichheiten der Amplifikation kann das Verfahren durch Normierung innerhalb des gleichen DNA-Abschnitts ausgleichen.

Die Behandlung von genomischer DNA mit Bisulfit ist eine effiziente und gut etablierte Methode um DNA-Methylierung zu analysieren. Dies setzt allerdings voraus, dass eine genügende DNA-Menge konvertiert wird. Bei einigen Materialien, die diagnostisch interessant sind, ist die Voraussetzung nicht sicher erfüllt. Beispiel dafür sind FFPE-Gewebe, freie zirkulierende DNA aus Körperflüssigkeiten, etc. Deswegen ist die Analyse nur der methylierten Fraktion sehr hilfreich. Dies ermöglicht die gut etablierte methylierungsspezifische Immunopräzipitation (MeDIP). Das MeDIP Verfahren basiert auf der Affinitätsreinigung von methylierter DNA unter Verwendung eines hoch spezifischen Antikörpers, der gegen 5-Methylcytosin (5-mC) gerichtet ist. Die Qualität der präzipitierten DNA ist abhängig von deren Herkunft. So tritt das Problem der genetischen Unähnlichkeit beispielsweise bei ungleich fragmentierter DNA (FFPE-Material) sowie Tumormaterial aller Arten ein. Der Einsatz präzipitierter DNA aus solchen Materialien bei der real time PCR kann das Ergebnis wegen vorexistierenden genetischen Unähnlichkeiten verfälschen. Das vorliegende Verfahren kann die eventuell vorliegende Unähnlichkeit umgehen, indem in einer real time PCR die Zielregion mit einer Region aus der nächsten Nähe des gleichen DNA-Abschnitts normiert und dadurch die Abweichung kompensiert und korrigiert wird.

### Figurenbeschreibung

**Fig. 1A** zeigt die relative Quantifizierung des FAM-Genlokus (auf Chr. X) mittels zwei verschiedenen FAM-sequenzspezifischen Primerpaaren (s1/as1 und s2/as2) und der Normierung auf den NKP46-Genlokus (auf Chr. 19) aus X monosomen und XX disomen Proben.
**Fig. 1B** zeigt die relative Quantifizierung des FAM-Genlokus (auf Chr. X) mittels zwei verschiedener FAM-sequenzspezifischer Primerpaare (s1/as1, linkes Diagramm und s2/as2, rechtes Diagramm) und der Idiolokalen Normierung auf den FAM-Genlokus (auf Chr. X) aus X monosomen und XX disomen Proben

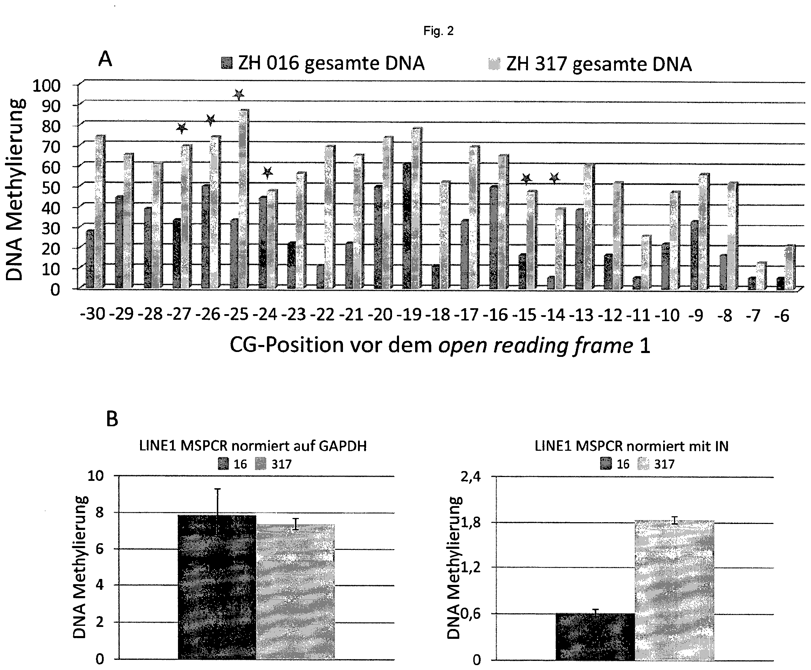

**Fig. 2A** gibt im Diagramm die DNA Methylierungsprofile der 436 Bp langen, CpG reichen LINE-1 Promotorregion wieder, wie sie mittels der genomischen Sequenzierung nach der Bisulfit-Konvertierung aus zwei urinären DNA-Proben von Harnblasenkarzinompatienten erhoben worden sind (ZH 016, ZH 317). Die Höhe eines Balkens gibt an, in wieviel Prozent der Sequenzen das jeweilige CpG Dinukleotid methyliert vorliegt. Die Ziffern auf der x-Achse geben die relative Positionierung der einzelnen CpG Dinukleotide zu dem ATG-Startkodon des ersten offenen Leserasters wieder. Die roten Sterne zeigen die CpG Dinukleotide an, die in dem Primerpaar vorhanden sind, welches für die MSPCR verwendet wurden (B).
**Fig. 2B** zeigt Bestimmungen der LINE1-DNA Methylierung mittels der MSPCR und den Primerpaaren, deren CpG Positionierung in A mittels roter Sternchen angezeigt ist. Das linke Diagramm zeigt ein MSPCR Resultat nach der Normierung auf GAPDH. Das rechte Diagramm zeigt das MSPCR Ergebnis nach der Idiolokalen Normierung.
**Fig.3a** : Normierung des JAM2-Gens auf das NKP46 Gen: Gezeigt wird die relative Menge des JAM2-Genlocus nach Quantifizierung mittels des spezifischen JAM2 Primerpaare s1/as1 und s3/as3 und Normierung auf den NKP46-Genlocus (chr. 19).
**Fig. 3b****:** Normierung des JAM2-Genlocus s1/as1 auf den JAM2-Genlocus s3/as3 Relative Menge des JAM2-Genlocus nach Quantifizierung mittels des JAM2 spezifischen Primerpaares s1/as1 und Normierung auf den benachbarten JAM2-Genlocus s3/as3.
**Fig. 3c****:** Normierung des JAM2-Genlocus s3/as3 auf den JAM2-Genlocus s1/as1Relative Mengen des JAM2-Genlocus nach Quantifizierung mittels des JAM2 spezifischen Primerpaares s3/as3 und Normierung auf den benachbarten JAM2-Genlocus s1/as1.

Die Erfindung wird durch die nachfolgenden Beispiele weiter erläutert.

### Beispiel 1: Fam

Als simulierte Aneuploidie wird das X-chromosomal gekoppelte FAM verwendet. FAM kommt wie das X-Chromosom in weiblicher DNA zweimal, in männlicher nur einmal vor. Es wurde mit dem konventionellen Normierungsverfahren, d. h. bezogen auf ein Gen (NKP46) normiert sowie mit dem erfindungsgemäßen Verfahren.

Genomische DNA wurde aus Blut verschiedener gesunder weiblicher und männlicher Probanden isoliert. Hierfür wurde das QIAamp DNA-Blood-Mini-Kit (Qiagen) verwendet. 200 ng DNA wurden anhand des EpiTect Bisulfite-Kit (Qiagen) nach den Anweisungen des Herstellers konvertiert. Methylierungsspezifische Primer wurden mittels des Programmes Oligo designed. 15 ng Bisulfit-konvertierte DNA wurde für die methylierungsspezifische PCR eingesetzt. Als Optimum für die Versuche in der MS-PCR ergaben sich für das Primerpaar s1/as1-FAM 57°C und das Primerpaar s2/as2-FAM 66°C. Es wurden 10 pmol Primer per Reaktion verwendet.

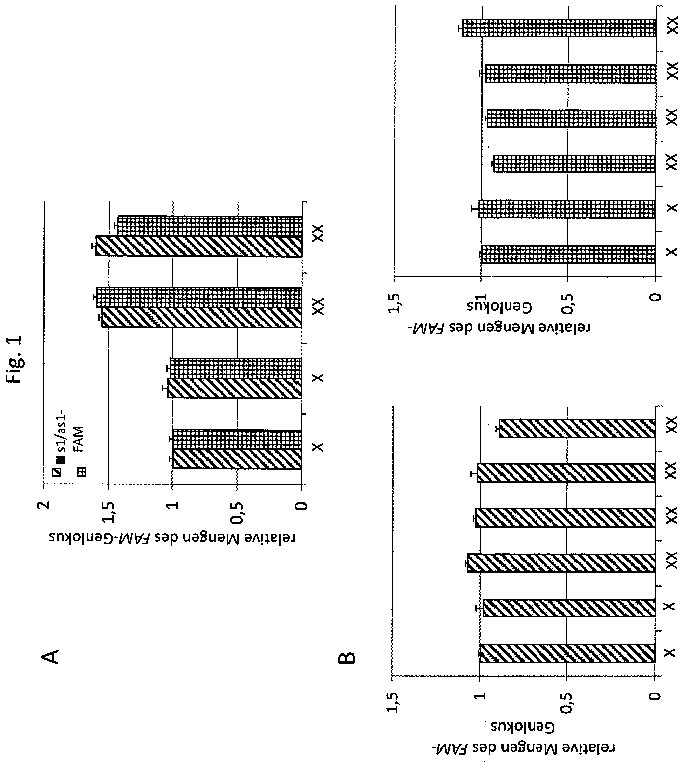

Ergebnis: Die konventionelle Normierung der FAM-Genloci auf den Genlocus des Gens NKP46 ergibt, dass der FAM-Genlocus im Mittel 1,5 fach bei den weiblichen gegenüber den männlichen Proben vorhanden ist (Figur 1a).

Nach dem erfindungsgemäßen Verfahren ergeben sich gleiche normierte Mengen der FAM-Genloci in den weiblichen wie in den männlichen Proben bei der Normierung des DNA-Abschnitts FAM über die Positionen s1/as1 und s2/as2 (Figur 1b).

### Beispiel 2: LINE1

Das DNA Methylierungsprofil der 436 Bp langen, CpG reichen LINE-1 Promotorregion wurde mittels der genomischen Sequenzierung nach der Bisulfit-Konvertierung aus zwei urinären DNA-Proben von Harnblasenkarzinompatienten erhoben (ZH 016, ZH 317) (2a). Zum Vergleich wurde im Anschluß die DNA Methylierung in ausgewählten Stellen des LINE-1 -DNA Methylierungsprofils mittels der MSPCR einmal nach der Normierung auf GAPDH zum Zweiten nach der Idiolokalen Normierung bestimmt (2b).

Ergebnis: Nur das MSPCR Ergebnis nach Normierung mit dem erfindungsgemäßen Verfahren ist mit dem Ergebnis der genomischen Sequenzierung übereinstimmend (2b rechtes Diagramm).

### Beispiel 3: JAM2

Als simulierte Aneuploidie wird das JAM2 Gen verwendet. Dieses liegt bei Gesunden diploid (2fach), bei Trisomie 21 triploid (3fach) vor. Das entspricht einer Aneuploidie im Verhältnis 1:1,5 von Gesundem zu Trisomie 21.

Es wurde mit dem konventionellen Normierungsverfahren, d. h. bezogen auf ein Gen (NKP46) normiert sowie mit dem erfindungsgemäßen Verfahren.

Genomische DNA wurde aus Blut verschiedener gesunder weiblicher und männlicher Probanden isoliert. Dazu wurde eine Probe verwendet, bei der eine Trisomie 21 vorliegt und cytogenetisch bestätigt. Hierfür wurde das QIAamp DNA-Blood-Mini-Kit (Qiagen) verwendet. 200 ng DNA wurden anhand des EpiTect Bisulfite-Kit (Qiagen) nach den Anweisungen des Herstellers konvertiert. Methylierungsspezifische Primer wurden mittels des Programmes Oligo designend. 15 ng Bisulfit-konvertierte DNA wurde für die methylierungsspezifische PCR eingesetzt. Als Optimum für die Versuche in der MS-PCR ergaben sich für das Primerpaar s1/as1-JAM und das Primerpaar s3/as3-JAM mit Annealingstemperatur von 67°C. Es wurden 10 pmol Primer per Reaktion verwendet.

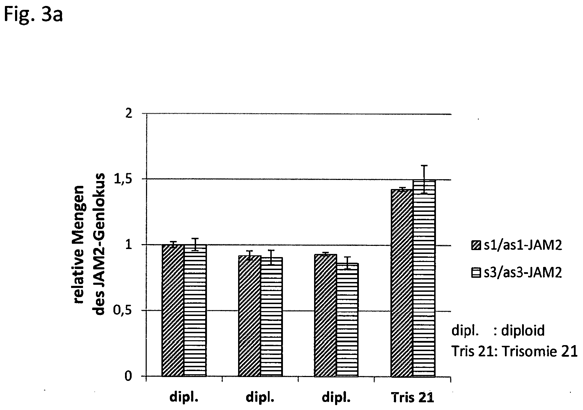

Ergebnis: Die konventionelle Normierung von JAM2 auf das Gen NKP46 zeigt mindestens die 1,5 fache relative Menge bei Trisomie 21 gegenüber den "gesunden" - diploiden - Proben (Figur 3a).

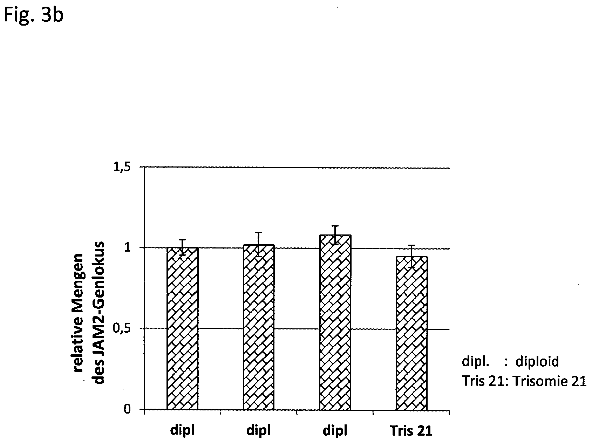

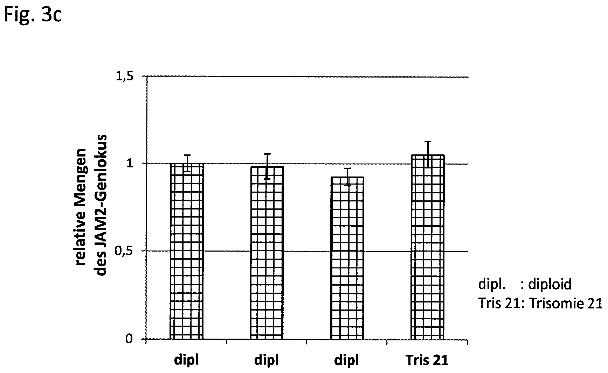

Nach dem erfindungsgemäßen Verfahren ergeben sich annähernd gleiche relative Mengen der JAM2-Genloci in den "gesunden" (diploiden) wie in der Trisomie 21-Probe z.B. bei der Normierung von JAM2 s1/as1 auf JAM s3/as3 (Figur 3b,3c).

### Beispiel 4: RASSF1

Als simulierte Proben wurden die Blasenkrebszelllinien HT1376 und Sw1710 verwendet. Die Zelllinie HT1376 hat einen Zugewinn des Chromosoms 3, in dem das RASSF1 Gen liegt und hat einen Verlust des 12p12.3-pter Bereichs, in dem das GAPDH Gen liegt (Hurst et al., Oncogene 2004; 23(12):2250-63). Die DNA Methylierung des RASSF1 5' Bereichs ist in HT1376 vergleichbar hoch wie in der Harnblasenkarzinomzelllinie Sw1710 (Neuhausen et al., Cancer Biol Ther. 2006 Aug; 5(8):993-1001.). Dazu wurde mit dem konventionellen Normierungsverfahren, d. h. bezogen auf ein Housekeeping-Gen (GAPDH) normiert sowie mit dem erfindungsgemäßen Verfahren.

Genomische DNA wurde aus gut beschriebenen Zelllinien isoliert. Hierfür wurde das QIAamp DNA-Blood-Mini-Kit (Qiagen) verwendet. 200 ng DNA wurden anhand des EpiTect Bisulfite-Kit (Qiagen) nach den Anweisungen des Herstellers konvertiert. Methylierungsspezifische Primer wurden mittels des Programmes Oligo designend. 15 ng Bisulfit-konvertierte DNA wurde für die methylierungsspezifische PCR eingesetzt. Das Optimum des Primerpaares RASSF1 Kon1 (Kontrollprimerpaar) lag bei 54 °C und für RASSF1 MS (methylspezifisches Primerpaar) bei 58°C. Es wurden 10 pmol Primer per Reaktion verwendet.

### Konventionelle Normierung

Proben von Sw1710 und HT1376 umfassend 15 ng DNA wurden mit den Primern s/as RASSF1 in parallelen Experimenten amplifiziert. Es ergaben sich gemittelte ct-Werte von
Sw1710: 27,16
HT1376: 25,86

Zur Normierung wurden die Primer s1/as für GAPDH verwendet; die gemessenen gemittelten ct-Werte betrugen
Sw1710: 26,15
HT1376: 28,74

Die Differenz (ΔCT) zwischen dem ct-Wert für RASSF1 und der GAPDH Kontrolle betrug
Sw1710: 1,01
HT1376: -2,88

Die Zelllinie Sw1710 wurde als Standard angesehen. Die Differenz der Δct-Werte (ΔΔCt) betrug dann -3,90. Das Verhältnis des Vorkommens der methylierten Sequenz ergibt sich dann als 2-ΔΔt = 14,9.

### Erfindungsgemäße Normierung

Wie bei der konventionellen Normierung wurden die ct-Werte der beiden Zellen bestimmt. Es ergaben sich die ct-Werte von
Sw1710: 27,16
HT1376: 25,86

In diesem Fall wurde aber als Kontrolle der Primer s1/as1 RASSF1 Kontrolle verwendet. Hiermit ergaben sich ct-Mittelwerte von
Sw1710: 23,7
HT1376: 24,70

Die Differenz (ΔCT) zwischen dem ct-Wert für RASSF1 und der RASSF1 Kontrolle betrug
Sw1710: 3,46
HT1376: 1,16

Die Differenz der beiden ct-Werte (ΔΔCt) betrug -2,31. Damit ergab sich eine Differenz des Vorkommens von 2-ΔΔct von 4,95.

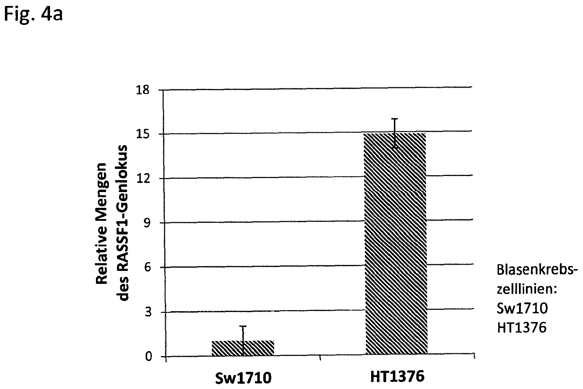

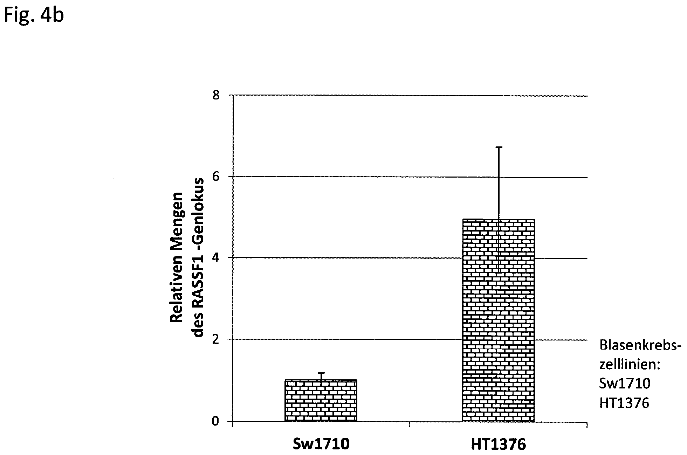

Ergebnis: Nach der konventionellen Normierung beträgt die Methylierung von RASSF1 in der Blasenzelllinie HT1376 das 15 Fache gegenüber der in Sw1710 (Figur 4a). Nach dem erfindungsgemäßen Verfahren ist sie annähernd nur noch 5 fach so hoch (Figur 4b).

Die Anwendung der idiolokalen Normierung in der Methylierungsspezifischen PCR (MSPCR) führt zu einer Nivellierung der genetischen Unterschiede in den zu messenden DNA Proben, derartig, dass das Messergebnis nur auf den DNA Methylierungsgrad beruht.

### Beispiel 5: Verwendete Primer in den Beispielen 1 bis 4

**Tabelle 1: NKP46-Primersequenzen**

| Bezeichnung | DNA-Sequenz von 5' -> 3'-Richtung |
|---|---|
| s1 | CACGGCCTTTCTGTAAGCTCATGGTC |
| as1 | CAGCGTGATCCCATTCCCCTTCC |

**Tabelle 2: JAM2-Primersequenzen**

| Bezeichnung | DNA-Sequenz von 5' -> 3'-Richtung |
|---|---|
| s1 | TCCCCTCCCGACTCTCTGCTC |
| as1 | CCAGGCAGGAAGGGTAGAGGAAC |
| s3 | ATACATCCCCGTCCCCGAG |
| as3 | CTAGAGGGCGTGAAAACCAGAC |

**Tabelle 3: GAPDH-Primersequenzen**

| Bezeichnung | DNA-Sequenz von 5' -> 3'-Richtung |
|---|---|
| s1 | GGGACCTTCTTGCCTTGCTCTTGCT |
| as1 | TGAGTGTGGGATGGGAGGGTGCT |

**Tabelle 4: FAM- Primersequenzen**

| Bezeichnung | DNA-Sequenz von 5' -> 3'-Richtung |
|---|---|
| s1 | ATGATGCTTACCCTCTCCTTGTG |
| as1 | TCTCTGCTGCTTCTGGGTCC |
| s2 | CCAGAGAGGCGGACGAAGTT |
| as2 | TTTCACGCACTCCCATTTCTG |
| s3 | GGCTCACAAAGGATGGGGG |
| as3 | TAACTTCGTCCGCCTCTCTGG |

**Tabelle 5: RASSF1-Primersequenzen**

| Bezeichnung | DNA-Sequenz von 5' -> 3'-Richtung |
|---|---|
| s | GCGGTCGTCGTCGTTGTGGTC |
| as | GCCCAACGAATACCAACTCCCG |
| s Kon1 | TGTGTTTAGTTTTTTAGAGTAGGATTTG |
| as Kon1 | CTCTAAACCACTACCTCTAACACATC |

**Tabelle 6: konvertierte GAPDH-Primersequenzen**

| Bezeichnung | DNA-Sequenz von 5' -> 3'-Richtung |
|---|---|
| s KonvPr | GGTTTTTAGTGTTTAGTGTTTAGTGT |
| as KonvPr | CCCTTTCTTTCTTTCAAAAAC |

## Patentansprüche

1. Verfahren zur Bestimmung des relativen DNA-Methylierungsgrads, umfassend die Schritte:
(i) Bestimmung des normierten Methylierungsgrads eines oder mehrerer DNA-Abschnitts/e, ausgenommen repetitive Abschnitte, in einer Probe genomischer DNA, umfassend die Schritte
a) Quantitative Bestimmung der Gegenwart des DNA-Abschnitts in der Probe;
b) Quantitative Bestimmung des Vorliegens von zumindest einem differentiell methylierten C eines CpG Dinukleotids innerhalb des DNA-Abschnitts und
c) Bestimmung des normierten DNA-Methylierungsgrads des DNA-Abschnitts über die in den Schritten a) und b) bestimmten Werte für eine erste genomische DNA und eine zweite genomische DNA, wobei der DNA-Abschnitt ausgewählt ist aus einem oder mehreren der folgenden Gene:
FAM50B-Ups, PITPNM1-Ups, LOC100419707-Cor, NTAN1-Cor, RPL36AP39-Ups, MIR324-Ups, HMGN2P28-Ups, MIR5696-Cor, HMGN2P28-Cor, RPS27P20-Cor, MPP7-Ups, MYEF2-Ups, TMEM254-Ups, VWC2L-Ups, CFHR5-Cor, ZSCAN1-Cor, RPL3P10-Ups, MYEF2-Cor, TMSB4XP2-Cor, SCN8A-Ups, PPDPF-Cor, TRNAQ15-Cor, TCTN2-Cor, RAD21L1-Cor, JOSD2-Ups, ZNF586-Ups, C10orf47-Cor, KIR2DP1-Cor, MAGEB1-Ups, IFNA17-Ups, TRIM62-Ups, MAGEB1-Cor, CTSW-Ups, LOC100507165-Cor, MIR4692-Ups, PLIN2-Cor, RPL7AP64-Cor, HIGD1AP12-Cor, MST1-Cor, ITGAD-Ups, DAK-Cor, FAM66E-Cor, HMGB3-Ups, FAM90A24P-Cor, LOC283335-Cor, MIR338-Cor, ADORA2BP1-Cor, CALD1-Ups, POU5F1P6-Ups, ARHGEF1-Cor, CNFN-Ups, MAST1-Ups, SCDP1-Ups, OR6C2-Ups, HOXB-AS1-Cor, PPIGP1-Ups, RMI2-Cor, TIGD1-Cor,
LOC100421695-Cor, BRE-AS1-Cor, MIRLET7F2-Cor,
RNASEH2CP1-Cor, FAM90A24P-Ups, NAV2-AS5-Ups, SEPT3-Cor, RPS15AP36-Cor, KLRC1-Ups, KRT23-Ups,
RAD51D-Ups, RPL13P7-Ups, LOC100128048-Cor, OR4A42P-Cor, LOC100996515-Ups, ARHGEF39-Cor, CYP3A4-Ups, MTND5P20-Cor, TSGA10IP-Ups, RBBP9-Cor, RPL39P25-Cor, NTMT1-Cor, LOC101060199-Cor, ZNF491-Cor, CIT-Cor, TPTE2P3-Ups, LOC338817-Cor, AK3P3-Ups, LOC126860-Cor,
(ii) Bestimmung des relativen DNA-Methylierungsgrads über das Verhältnis der für die erste genomische DNA und die zweite genomische DNA bestimmten normierten Methylierungsgrade,
zur Diagnose einer Erkrankung, die mit einer veränderten DNA-Methylierung in Verbindung gebracht wird, wobei der relative Methylierungsgrad eine Hypermethylierung bedeutet und wobei die erste genomische DNA eine Referenzprobe ist und die zweite genomische DNA aus einer zu untersuchenden Probe stammt und die Erkrankung ein Prostata-Tumor ist.

2. Verfahren zur Bestimmung des relativen DNA-Methylierungsgrads, umfassend die Schritte
(i) Bestimmung des normierten Methylierungsgrads eines oder mehrerer DNA-Abschnitts/e, ausgenommen repetitive Abschnitte, in einer Probe genomischer DNA, umfassend die Schritte:
a) Quantitative Bestimmung der Gegenwart des DNA-Abschnitts in der Probe;
b) Quantitative Bestimmung des Vorliegens von zumindest einem differentiell methylierten C eines CpG Dinukleotids innerhalb des DNA-Abschnitts und
c) Bestimmung des normierten DNA-Methylierungsgrads des DNA-Abschnitts über die in den Schritten a) und b) bestimmten Werte für eine erste genomische DNA und eine zweite genomische DNA, wobei der DNA-Abschnitt ausgewählt ist aus einem oder mehreren der folgenden Gene:
WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, LOC401317-Cor, CRNKL1-Ups, MCCD1P2-Ups, DSG1-Cor, SLC22A24-Ups, ZNF26-Cor, NXPH4-Cor, MEN1-Ups, KDM3A-Cor, ARHGAP35-Cor, TRNAA2-Ups, EIF1AD-Ups, ACTN3-Cor, SPTY2D1-Cor, ASCL2-Ups, ZNF132-Cor, HMOX1-Cor, PPP1R2-Ups, WBSCR17-Cor, ARSH-Cor, ALDH7A1P3-Cor, NAGLU-Ups, ACRBP-Cor, ZNF304-Ups, MIR1972-2-Ups, TMEM33-Ups, LOC100419839-Ups, C1R-Cor, SNORD88B-Cor, NDUFB7-Cor, NTMT1-Ups, BRD4-Cor, LOC100131510-Cor, TIMM8BP2-Ups, KCNK1-Cor, RIMKLA-Cor, RPL12P42-Ups, PCGF6-Cor, LOC100419895-Cor, IZUMO2-Ups, PCBP3-Ups, ASS1P13-Cor, LOC100419848-Ups, LZTS1-AS1-Cor, LOC145757-Cor, PPFIA3-Cor, TRNAV20-Ups, LARP1P1-Cor, ZNF187-Ups, MIR4518-Cor, LOC100130394-Ups, ACPT-Cor, RPL23AP18-Cor, ODF2L-Cor, C16orf46-Ups, LOC100422241-Ups,
ZHX3-Ups, RPL23AP32-Ups, ANKRD12-Cor, CD27-AS1-Cor, TPI1-Ups, KCNG4-Ups, GUSBP5-Ups, LOC147670-Cor, HYDIN-Cor, PKD2L1-Ups, GRIFIN-Ups, MIR548AK-Cor, YBX2-Cor, HNRNPA3P4-Ups, ATG2A-Cor, CDC42EP5-Ups, HBG2-Ups, DDB1-Cor, FAM90A11P-Cor, RPL9P17-Ups, TBX5-AS1-Ups, ZNF415-Cor, AURKC-Ups, EPHA10-Cor, KBTBD11-Ups, LOC100996788-Ups, ORAI3-Ups, LOC100507530-Ups, VSTM2L-Ups, ACTR5-Cor, LOC284080-Cor, LOC100996366-Cor, MIR526A2-Cor, MIR516B2-Cor, LOC100130632-Cor, CIB3-Cor, CHST9-Cor, ZNF788-Ups, ANKRD16-Cor, EIF4E3-Cor, CTXN2-Cor, LOC100631378-Cor, RPS2P48-Cor, FAM87B-Ups, LOC644881-Cor, TRNAS7-Ups, LSMD1-Ups,
LOC390203-Ups, ARPC4-Cor, TAF6L-Ups, PROCR-Ups, RNU5B-4P-Cor, MIR548A2-Ups,-ASCL2-Cor, FOXRED1-Cor, MAPK8IP3-Cor, PIM1-Cor, HMGN2P2-Ups,
MIR3972-Ups, LHFPL3-AS1-Ups, ZNF528-Ups, RPS15AP32-Ups, MIR516B2-Ups, FUT5-Cor, GPX1-Cor, C7orf65-Cor, RNY5P6-Ups, SYDE1-Cor, LOC389120-Ups, CDK2AP1-Cor, MIR518B-Cor, LOC100506753-Cor, REP15-Ups, EIF4G3-Ups, HIATL2-Ups, ZNF534-Cor, LOC115110-Cor, ATXN7L1-Ups, LRRC3C-Ups, VTI1BP3-Cor, LOC100507206-Ups, PUS1-Cor, SRP14P1-Ups, ODC1-Ups, RPL30P4-Ups, ZNF229-Cor, SIGLEC7-Ups, LOC654780-Cor, TPBGL-Cor,
EHD1-Ups, TCEB3-Cor, KCNG4-Cor, SNORD5-Cor, CTXN2-Cor, PITPNM1-Ups, LDHB-Cor, LY6G5C-Ups, ID3-Cor, PELI3-Cor, MIR526A2-Cor, RPS29P24-Ups, IMPDH1P5-Ups, VWC2L-Cor, FAM71E2-Ups, RPL21P4-Ups, KCNQ4-Ups, ZNF512-Cor, ANKRD16-Cor, DHX8-Cor, STARD3-Cor, ZSCAN23-Ups, LOC100130632-Cor, KLF2-Cor, VSTM5-Ups, LOC151174-Cor, PGBD1-Ups, APPL1-Ups, COX6A1P2-Ups, ZNF775-Ups, SLC16A6-Cor, RPL9P29-Cor, IFT74-Cor, LOC100420668-Cor, IGSF21-Ups, PCBP3-Cor,
(ii) Bestimmung des relativen DNA-Methylierungsgrads über das Verhältnis der für die erste und die zweite DNA bestimmten Methylierungsgrade,
zur Diagnose einer Erkrankung, die mit einer veränderten DNA-Methylierung in Verbindung gebracht wird, wobei der relative Methylierungsgrad eine Hypomethylierung bedeutet und wobei die erste genomische DNA eine Referenzprobe ist und die zweite genomische DNA aus einer zu untersuchenden Probe stammt und die Erkrankung ein Prostata-Tumor ist.

## Claims

1. Method for determining the relative degree of DNA methylation, comprising the steps of:
(i) determining the normalized degree of methylation of one or more DNA segments, excluding repetitive segments, in a sample of genomic DNA, comprising the steps of:
a) quantitatively determining the presence of the one or more DNA segments in the sample;
b) quantitatively determining the presence of at least one differentially methylated C of a CpG dinucleotide within the one or more DNA segments, and
c) determining the normalized degree of DNA methylation of the one or more DNA segments using the values determined in steps a) and b) for first genomic DNA and a second genomic DNA, wherein the one or more DNA segments is/are selected from one or more of the following genes:
FAM50B-Ups, PITPNM1-Ups, LOC100419707-Cor, NTAN1-Cor, RPL36AP39-Ups, MIR324-Ups, HMGN2P28-Ups, MIR5696-Cor, HMGN2P28-Cor, RPS27P20-Cor, MPP7-Ups, MYEF2-Ups, TMEM254-Ups, VWC2L-Ups, CFHR5-Cor, ZSCAN1-Cor, RPL3P10-Ups, MYEF2-Cor, TMSB4XP2-Cor, SCN8A-Ups, PPDPF-Cor, TRNAQ15-Cor, TCTN2-Cor, RAD21L1-Cor, JOSD2-Ups, ZNF586-Ups, C10orf47-Cor, KIR2DP1-Cor, MAGEB1-Ups, IFNA17-Ups, TRIM62-Ups, MAGEB1-Cor, CTSW-Ups, LOC100507165-Cor, MIR4692-Ups, PLIN2-Cor, RPL7AP64-Cor, HIGD1AP12-Cor, MST1-Cor, ITGAD-Ups, DAK-Cor, FAM66E-Cor, HMGB3-Ups, FAM90A24P-Cor, LOC283335-Cor, MIR338-Cor, ADORA2BP1-Cor, CALD1-Ups, POU5F1P6-Ups, ARHGEF1-Cor, CNFN-Ups, MAST1-Ups, SCDP1-Ups, OR6C2-Ups, HOXB-AS1-Cor, PPIGP1-Ups, RMI2-Cor, TIGD1-Cor,
LOC100421695-Cor, BRE-AS1-Cor, MIRLET7F2-Cor,
RNASEH2CP1-Cor, FAM90A24P-Ups, NAV2-AS5-Ups, SEPT3-Cor, RPS15AP36-Cor, KLRC1-Ups, KRT23-Ups,
RAD51D-Ups, RPL13P7-Ups, LOC100128048-Cor, OR4A42P-Cor, LOC100996515-Ups, ARHGEF39-Cor, CYP3A4-Ups, MTND5P20-Cor, TSGA10IP-Ups, RBBP9-Cor, RPL39P25-Cor, NTMT1-Cor, LOC101060199-Cor, ZNF491-Cor, CIT-Cor, TPTE2P3-Ups, LOC338817-Cor, AK3P3-Ups, LOC126860-Cor,
(ii) determining the relative degree of DNA methylation using the relationship between the normalized degrees of methylation determined for the first genomic DNA and for the second genomic DNA,
to diagnose a disease that is associated with altered DNA methylation, wherein the relative degree of methylation means a hypermethylation and wherein the first genomic DNA is a reference sample and the second genomic DNA originates from a sample to be examined and the disease is a prostate tumor.

2. Method for determining the relative degree of DNA methylation, comprising the steps of:
(i) determining the normalized degree of methylation of one or more DNA segments, excluding repetitive segments, in a sample of genomic DNA, comprising the steps of:
a) quantitatively determining the presence of the one or more DNA segments in the sample;
b) quantitatively determining the presence of at least one differentially methylated C of a CpG dinucleotide within the one or more DNA segments, and
c) determining the normalized degree of DNA methylation of the one or more DNA segments using the values determined in steps a) and b) for first genomic DNA and second genomic DNA, wherein the one or more DNA segments is/are selected from one or more of the following genes:
WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, LOC401317-Cor, CRNKL1-Ups, MCCD1P2-Ups, DSG1-Cor, SLC22A24-Ups, ZNF26-Cor, NXPH4-Cor, MEN1-Ups, KDM3A-Cor, ARHGAP35-Cor, TRNAA2-Ups, EIF1AD-Ups, ACTN3-Cor, SPTY2D1-Cor, ASCL2-Ups, ZNF132-Cor, HMOX1-Cor, PPP1R2-Ups, WBSCR17-Cor, ARSH-Cor, ALDH7A1P3-Cor, NAGLU-Ups, ACRBP-Cor, ZNF304-Ups, MIR1972-2-Ups, TMEM33-Ups, LOC100419839-Ups, C1R-Cor, SNORD88B-Cor, NDUFB7-Cor, NTMT1-Ups, BRD4-Cor, LOC100131510-Cor, TIMM8BP2-Ups, KCNK1-Cor, RIMKLA-Cor, RPL12P42-Ups, PCGF6-Cor, LOC100419895-Cor, IZUMO2-Ups, PCBP3-Ups, ASSIP13-Cor, LOC100419848-Ups, LZTS1-AS1-Cor, LOC145757-Cor, PPF1A3-Cor, TRNAV20-Ups, LARP1P1-Cor, ZNF187-Ups, MIR4518-Cor, LOC100130394-Ups, ACPT-Cor, RPL23AP18-Cor, ODF2L-Cor, C16orf46-Ups, LOC100422241-Ups,
ZHX3-Ups, RPL23AP32-Ups, ANKRD12-Cor, CD27-AS1-Cor, TPI1-Ups, KCNG4-Ups, GUSBP5-Ups, LOC147670-Cor, HYDIN-Cor, PKD2L1-Ups, GRIFIN-Ups, MIR548AK-Cor, YBX2-Cor, HNRNPA3P4-Ups, ATG2A-Cor, CDC42EP5-Ups, HBG2-Ups, DDB1-Cor, FAM90A11P-Cor, RPL9P17-Ups, TBX5-AS1-Ups, ZNF415-Cor, AURKC-Ups, EPHA10-Cor, KBTBD11-Ups, LOC100996788-Ups, ORAI3-Ups, LOC100507530-Ups, VSTM2L-Ups, ACTR5-Cor, LOC284080-Cor, LOC100996366-Cor, MIR526A2-Cor, MIR516B2-Cor, LOC100130632-Cor, CIB3-Cor, CHST9-Cor, ZNF788-Ups, ANKRD16-Cor, EIF4E3-Cor, CTXN2-Cor, LOC100631378-Cor, RPS2P48-Cor, FAM87B-Ups, LOC644881-Cor, TRNAS7-Ups, LSMD1-Ups,
LOC390203-Ups, ARPC4-Cor, TAF6L-Ups, PROCR-Ups, RNU5B-4P-Cor, M1R548A2-Ups,-ASCL2-Cor, FOXRED1-Cor, MAPK8IP3-Cor, PIM1-Cor, HMGN2P2-Ups, MIR3972-Ups, LHFPL3-AS1-Ups, ZNF528-Ups, RPS15AP32-Ups, MIR516B2-Ups, FUT5-Cor, GPX1-Cor, C7orf65-Cor, RNY5P6-Ups, SYDE1-Cor, LOC389120-Ups, CDK2AP1-Cor, MIR518B-Cor, LOC100506753-Cor, REP15-Ups, EIF4G3-Ups, HIATL2-Ups, ZNF534-Cor, LOC115110-Cor, ATXN7L1-Ups, LRRC3C-Ups, VTI1BP3-Cor, LOC100507206-Ups, PUS1-Cor, SRP14P1-Ups, ODC1-Ups, RPL30P4-Ups, ZNF229-Cor, SIGLEC7-Ups, LOC654780-Cor, TPBGL-Cor,
EHD1-Ups, TCEB3-Cor, KCNG4-Cor, SNORD5-Cor, CTXN2-Cor, PITPNM1-Ups, LDHB-Cor, LY6G5C-Ups, ID3-Cor, PELI3-Cor, MIR526A2-Cor, RPS29P24-Ups, IMPDH1P5-Ups, VWC2L-Cor, FAM71E2-Ups, RPL21P4-Ups, KCNQ4-Ups, ZNF512-Cor, ANKRD16-Cor, DHX8-Cor, STARD3-Cor, ZSCAN23-Ups, LOC100130632-Cor, KLF2-Cor, VSTM5-Ups, LOC151174-Cor, PGBD1-Ups, APPL1-Ups, COX6A1P2-Ups, ZNF775-Ups, SLC16A6-Cor, RPL9P29-Cor, IFT74-Cor, LOC100420668-Cor, IGSF21-Ups, PCBP3-Cor,
(ii) determining the relative degree of DNA methylation using the relationship between the degrees of methylation determined for the first DNA and for the second DNA,
to diagnose a disease that is associated with altered DNA methylation, wherein the relative degree of methylation means a hypomethylation and wherein the first genomic DNA is a reference sample and the second genomic DNA originates from a sample to be examined and the disease is a prostate tumor.

## Revendications

1. Procédé permettant de déterminer le degré de méthylation relatif d'ADN, comprenant les étapes de :
(i) détermination du degré de méthylation normalisé d'un ou plusieurs segments d'ADN, à l'exception de segments répétitifs, dans un échantillon d'ADN génomique, comprenant les étapes de
a) détermination quantitative de la présence du segment d'ADN dans l'échantillon ;
b) détermination quantitative de la présence d'au moins un C différentiellement méthylé d'un dinucléotide CpG à l'intérieur du segment d'ADN et
c) détermination du degré de méthylation normalisé d'ADN du segment d'ADN à l'aide des valeurs déterminées aux étapes a) et b) pour un premier ADN génomique et un second ADN génomique, le segment d'ADN étant choisi parmi un ou plusieurs des gènes suivants :
FAM50B-Ups, PITPNM1-Ups, LOC100419707-Cor, NTAN1-Cor, RPL36AP39-Ups, MIR324-Ups, HMGN2P28-Ups, MIR5696-Cor, HMGN2P28-Cor, RPS27P20-Cor, MPP7-Ups, MYEF2-Ups, TMEM254-Ups, VWC2L-Ups, CFHR5-Cor, ZSCAN1-Cor, RPL3P10-Ups, MYEF2-Cor, TMSB4XP2-Cor, SCN8A-Ups, PPDPF-Cor, TRNAQ15-Cor, TCTN2-Cor, RAD21L1-Cor, JOSD2-Ups, ZNF586-Ups, C10orf47-Cor, KIR2DP1-Cor, MAGEB1-Ups, IFNA17-Ups, TRIM62-Ups, MAGEB1-Cor, CTSW-Ups, LOC100507165-Cor, MIR4692-Ups, PLIN2-Cor, RPL7AP64-Cor, HIGD1AP12-Cor, MST1-Cor, ITGAD-Ups, DAK-Cor, FAM66E-Cor, HMGB3-Ups, FAM90A24P-Cor, LOC283335-Cor, MIR338-Cor, ADORA2BP1-Cor, CALD1-Ups, POU5F1P6-Ups, ARHGEF1-Cor, CNFN-Ups, MAST1-Ups, SCDP1-Ups, OR6C2-Ups, HOXB-AS1-Cor, PPIGP1-Ups, RMI2-Cor, TIGD1-Cor,
LOC100421695-Cor, BRE-AS1-Cor, MIRLET7F2-Cor,
RNASEH2CP1-Cor, FAM90A24P-Ups, NAV2-AS5-Ups, SEPT3-Cor, RPS15AP36-Cor, KLRC1-Ups, KRT23-Ups,
RAD51D-Ups, RPL13P7-Ups, LOC100128048-Cor, OR4A42P-Cor, LOC100996515-Ups, ARHGEF39-Cor, CYP3A4-Ups, MTND5P20-Cor, TSGA10IP-Ups, RBBP9-Cor, RPL39P25-Cor, NTMT1-Cor, LOC101060199-Cor, ZNF491-Cor, CIT-Cor, TPTE2P3-Ups, LOC338817-Cor, AK3P3-Ups, LOC126860-Cor,
(ii) détermination du degré de méthylation relatif d'ADN à l'aide du rapport des degrés de méthylation normalisés déterminés pour le premier ADN génomique et le second ADN génomique,
pour diagnostiquer une maladie associée à une méthylation modifiée de l'ADN, le degré de méthylation relatif indiquant une hyperméthylation et le premier ADN génomique étant un échantillon de référence et le second ADN génomique provenant d'un échantillon à examiner et la maladie étant une tumeur de la prostate.

2. Procédé permettant de déterminer le degré de méthylation relatif d'ADN, comprenant les étapes de
(i) détermination du degré de méthylation normalisé d'un ou plusieurs segments d'ADN, à l'exception de segments répétitifs, dans un échantillon d'ADN génomique, comprenant les étapes de :
a) détermination quantitative de la présence du segment d'ADN dans l'échantillon ;
b) détermination quantitative de la présence d'au moins un C différentiellement méthylé d'un dinucléotide CpG à l'intérieur du segment d'ADN et
c) détermination du degré de méthylation normalisé d'ADN du segment d'ADN à l'aide des valeurs déterminées aux étapes a) et b) pour un premier ADN génomique et un second ADN génomique, le segment d'ADN étant choisi parmi un ou plusieurs des gènes suivants :
WBP11-Cor, LOC100996630-Cor, B4GALNT4-Ups, ALOX15P1-Cor, TRNAR3-Ups, UBXN6-Ups, KIR2DL3-Cor, TAF6L-Cor, OR52P2P-Cor, LOC401317-Cor, CRNKL1-Ups, MCCD1P2-Ups, DSG1-Cor, SLC22A24-Ups, ZNF26-Cor, NXPH4-Cor, MEN1-Ups, KDM3A-Cor, ARHGAP35-Cor, TRNAA2-Ups, EIF1AD-Ups, ACTN3-Cor, SPTY2D1-Cor, ASCL2-Ups, ZNF132-Cor, HMOX1-Cor, PPP1R2-Ups, WBSCR17-Cor, ARSH-Cor, ALDH7A1P3-Cor, NAGLU-Ups, ACRBP-Cor, ZNF304-Ups, MIR1972-2-Ups, TMEM33-Ups, LOC100419839-Ups, C1R-Cor, SNORD88B-Cor, NDUFB7-Cor, NTMT1-Ups, BRD4-Cor, LOC100131510-Cor, TIMM8BP2-Ups, KCNK1-Cor, RIMKLA-Cor, RPL12P42-Ups, PCGF6-Cor, LOC100419895-Cor, IZUMO2-Ups, PCBP3-Ups, ASS1P13-Cor, LOC100419848-Ups, LZTS1-AS1-Cor, LOC145757-Cor, PPFIA3-Cor, TRNAV20-Ups, LARP1P1-Cor, ZNF187-Ups, MIR4518-Cor, LOC100130394-Ups, ACPT-Cor, RPL23AP18-Cor, ODF2L-Cor, C16orf46-Ups, LOC100422241-Ups,
ZHX3-Ups, RPL23AP32-Ups, ANKRD12-Cor, CD27-AS1-Cor, TPI1-Ups, KCNG4-Ups, GUSBP5-Ups, LOC147670-Cor, HYDIN-Cor, PKD2L1-Ups, GRIFIN-Ups, MIR548AK-Cor, YBX2-Cor, HNRNPA3P4-Ups, ATG2A-Cor, CDC42EP5-Ups, HBG2-Ups, DDB1-Cor, FAM90A11P-Cor, RPL9P17-Ups, TBX5-AS1-Ups, ZNF415-Cor, AURKC-Ups, EPHA10-Cor, KBTBD11-Ups, LOC100996788-Ups, ORAI3-Ups, LOC100507530-Ups, VSTM2L-Ups, ACTR5-Cor, LOC284080-Cor, LOC100996366-Cor, MIR526A2-Cor, MIR516B2-Cor, LOC100130632-Cor, CIB3-Cor, CHST9-Cor, ZNF788-Ups, ANKRD16-Cor, EIF4E3-Cor, CTXN2-Cor, LOC100631378-Cor, RPS2P48-Cor, FAM87B-Ups, LOC644881-Cor, TRNAS7-Ups, LSMD1-Ups,
LOC390203-Ups, ARPC4-Cor, TAF6L-Ups, PROCR-Ups, RNU5B-4P-Cor, MIR548A2-Ups, ASCL2-Cor, FOXRED1-Cor, MAPK8IP3-Cor, PIM1-Cor, HMGN2P2-Ups, MIR3972-Ups, LHFPL3-AS1-Ups, ZNF528-Ups, RPS15AP32-Ups, MIR516B2-Ups, FUT5-Cor, GPX1-Cor, C7orf65-Cor, RNY5P6-Ups, SYDE1-Cor, LOC389120-Ups, CDK2AP1-Cor, MIR518B-Cor, LOC100506753-Cor, REP15-Ups, EIF4G3-Ups, HIATL2-Ups, ZNF534-Cor, LOC115110-Cor, ATXN7L1-Ups, LRRC3C-Ups, VTI1BP3-Cor, LOC100507206-Ups, PUS1-Cor, SRP14P1-Ups, ODC1-Ups, RPL30P4-Ups, ZNF229-Cor, SIGLEC7-Ups, LOC654780-Cor, TPBGL-Cor,
EHD1-Ups, TCEB3-Cor, KCNG4-Cor, SNORD5-Cor, CTXN2-Cor, PITPNM1-Ups, LDHB-Cor, LY6G5C-Ups, ID3-Cor, PELI3-Cor, MIR526A2-Cor, RPS29P24-Ups, IMPDH1P5-Ups, VWC2L-Cor, FAM71E2-Ups, RPL21P4-Ups, KCNQ4-Ups, ZNF512-Cor, ANKRD16-Cor, DHX8-Cor, STARD3-Cor, ZSCAN23-Ups, LOC100130632-Cor, KLF2-Cor, VSTM5-Ups, LOC151174-Cor, PGBD1-Ups, APPL1-Ups, COX6A1P2-Ups, ZNF775-Ups, SLC16A6-Cor, RPL9P29-Cor, IFT74-Cor, LOC100420668-Cor, IGSF21-Ups, PCBP3-Cor,
(ii) détermination du degré de méthylation relatif d'ADN à l'aide du rapport des degrés de méthylation déterminés pour le premier et le second ADN,
pour le diagnostic d'une maladie associée à une méthylation modifiée de l'ADN, le degré de méthylation relatif indiquant une hypométhylation et le premier ADN génomique étant un échantillon de référence et le second ADN génomique provenant d'un échantillon à examiner et la maladie étant une tumeur de la prostate.


### SEQUENCE LISTING

<110> Heinrich-Heine-Universität Düsseldorf
<120> Idiolokale Normierung
<130> 131540WO
<160> 20
<170> PatentIn version 3.5
<210> 1
   <211> 26
   <212> DNA
   <213> Artificial Sequence
<220>
   <223> NKP46-Primer s1
<400> 1
   cacggccttt ctgtaagctc atggtc 26
<210> 2
   <211> 23
   <212> DNA
   <213> Artificial Sequence
<220>
   <223> NKP46-Primer as1
<400> 2
   cagcgtgatc ccattcccct tcc 23
<210> 3
   <211> 21
   <212> DNA
   <213> Artificial Sequence
<220>
   <223> JAM2-Primer s1
<400> 3
   tcccctcccg actctctgct c 21
<210> 4
   <211> 23
   <212> DNA
   <213> Artificial Sequence
<220>
   <223> JAM2-Primer as1
<400> 4
   ccaggcagga agggtagagg aac 23
<210> 5
   <211> 19
   <212> DNA
   <213> Artificial Sequence
<220>
   <223> JAM2-Primer s3
<400> 5
   atacatcccc gtccccgag 19
<210> 6
   <211> 22
   <212> DNA
   <213> Artificial Sequence
<220>
   <223> JAM2-Primer as3
<400> 6
   ctagagggcg tgaaaaccag ac 22
<210> 7
   <211> 25
   <212> DNA
   <213> Artificial Sequence
<220>
   <223> GAPDH-Primer s1
<400> 7
   gggaccttct tgccttgctc ttgct 25
<210> 8
   <211> 23
   <212> DNA
   <213> Artificial Sequence
<220>
   <223> GAPDH-Primer as1
<400> 8
   tgagtgtggg atgggagggt gct 23
<210> 9
   <211> 23
   <212> DNA
   <213> Artificial Sequence
<220>
   <223> FAM- Primer s1
<400> 9
   atgatgctta ccctctcctt gtg 23
<210> 10
   <211> 20
   <212> DNA
   <213> Artificial Sequence
<220>
   <223> FAM- Primer as1
<400> 10
   tctctgctgc ttctgggtcc 20
<210> 11
   <211> 20
   <212> DNA
   <213> Artificial Sequence
<220>
   <223> FAM- Primer s2
<400> 11
   ccagagaggc ggacgaagtt 20
<210> 12
   <211> 21
   <212> DNA
   <213> Artificial Sequence
<220>
   <223> FAM- Primer as2
<400> 12
   tttcacgcac tcccatttct g 21
<210> 13
   <211> 19
   <212> DNA
   <213> Artificial Sequence
<220>
   <223> FAM- Primer s3
<400> 13
   ggctcacaaa ggatggggg 19
<210> 14
   <211> 21
   <212> DNA
   <213> Artificial Sequence
<220>
   <223> FAM- Primer as3
<400> 14
   taacttcgtc cgcctctctg g 21
<210> 15
   <211> 21
   <212> DNA
   <213> Artificial Sequence
<220>
   <223> RASSF1-Primer s
<400> 15
   gcggtcgtcg tcgttgtggt c 21
<210> 16
   <211> 22
   <212> DNA
   <213> Artificial Sequence
<220>
   <223> RASSF1-Primer as
<400> 16
   gcccaacgaa taccaactcc cg 22
<210> 17
   <211> 28
   <212> DNA
   <213> Artificial Sequence
<220>
   <223> RASSF1-Primer s Kon1
<400> 17
   tgtgtttagt tttttagagt aggatttg 28
<210> 18
   <211> 26
   <212> DNA
   <213> Artificial Sequence
<220>
   <223> RASSF1-Primer as Kon1
<400> 18
   ctctaaacca ctacctctaa cacatc 26
<210> 19
   <211> 26
   <212> DNA
   <213> Artificial Sequence
<220>
   <223> GAPDH-Primer s KonvPr
<400> 19
   ggtttttagt gtttagtgtt tagtgt 26
<210> 20
   <211> 21
   <212> DNA
   <213> Artificial Sequence
<220>
   <223> GAPDH-Primer as KonvPr
<400> 20
   ccctttcttt ctttcaaaaa c 21